(19) Europäisches Patentamt
European Patent Office
Office européen des brevets

(11) **EP 3 113 846 B1**

(12) **EUROPÄISCHE PATENTSCHRIFT**

(45) Veröffentlichungstag und Bekanntmachung des Hinweises auf die Patenterteilung:
**24.07.2019 Patentblatt 2019/30**

(21) Anmeldenummer: **15703605.4**

(22) Anmeldetag: **11.02.2015**

(51) Int Cl.:
*A61Q 1/00* (2006.01)       *A61Q 15/00* (2006.01)
*A61Q 17/04* (2006.01)       *A61Q 19/00* (2006.01)
*A61K 8/85* (2006.01)       *B01F 17/00* (2006.01)
*C07C 69/33* (2006.01)       *A61K 8/86* (2006.01)

(86) Internationale Anmeldenummer:
**PCT/EP2015/052812**

(87) Internationale Veröffentlichungsnummer:
**WO 2015/132053 (11.09.2015 Gazette 2015/36)**

(54) **POLYGLYCERINESTER MIT BESONDERER OLIGOMERENVERTEILUNG DES POLYGLYCERINS**

POLYGLYCEROL ESTERS FEATURING A PARTICULAR OLIGOMER DISTRIBUTION IN THE POLYGLYCEROL

ESTER DE POLYGLYCÉRINE PRÉSENTANT UNE DISTRIBUTION PARTICULIÈRE DES OLIGOMÈRES DE POLYGLYCÉRINE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.03.2014 DE 102014203868**

(43) Veröffentlichungstag der Anmeldung:
**11.01.2017 Patentblatt 2017/02**

(73) Patentinhaber: **Evonik Degussa GmbH**
**45128 Essen (DE)**

(72) Erfinder:
• **FRIEDRICH, Achim**
**45529 Hattingen (DE)**
• **MEYER, Jürgen**
**45134 Essen (DE)**

• **VON HOF, Jan Marian**
**45138 Essen (DE)**
• **SPRINGER, Oliver**
**46485 Wesel (DE)**
• **MUSS, Peter**
**45359 Essen (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
**EP-A2- 2 363 387       EP-A2- 2 705 832**
**WO-A1-2008/103289       WO-A1-2012/127129**
**US-A1- 2012 156 271**

**Beschreibung**

Gebiet der Erfindung

Gegenstand der Erfindung sind neue Polyglycerinester mit besonderer Oligomerenverteilung des Polyglycerins sowie deren Verwendung in insbesondere kosmetischen Formulierungen.

Stand der Technik

In den letzten Jahren ist auf dem Kosmetikmarkt ein starker Trend zu Produkten auf Basis nachwachsender Rohstoffe zu verfolgen. Um diesen erfüllen zu können, ist es notwendig, leistungsstarke Emulgatoren auf Basis nachwachsender Rohstoffe anbieten zu können. Gängige Emulgatoren in der Kosmetik enthalten als hydrophile Gruppen oft Polyethylenglykolgruppen (PEG), die durch Polymerisation von petrochemisch gewonnenem Ethylenoxid hergestellt werden können. Da in möglichst natürlichen Formulierungen alle eingesetzten Rohstoffe aus nachwachsenden Quellen stammen sollten, sind PEG-haltige Emulgatoren in solchen Formulierungen nicht erwünscht.
Polyglycerinester sind eine bevorzugte PEG-freie Alternative für kosmetische Emulgatoren auf Basis nachwachsender Rohstoffe.
Der Einsatz von Polyglycerinestern in Kosmetika als Emulgator ist eine altbekannte Technik. So beschreibt die JP 2003104852 die Verwendung eines bei Raumtemperatur festen Polyglycerin-Behenat-Stearats in Haarpflegeprodukten.
Die JP 2010178723 beschreibt die Verwendung von Polyglycerinpartialestern von Behensäure in Verbindung mit anderen, kürzerkettigen Fettsäuren als Teig-Verbesserer in Backwaren.
JP 2006055029 beschreibt die Verwendung von Triglycerin-Monobehenate-Monostearat in Pflanzenschutzanwendungen.
EP2363387 beschreibt die Herstellung von Polyglycerin-Carbonsäure-Partialestern und deren Verwendung als Emulgatoren in Kosmetik.
Mit den im Stand der Technik beschriebenen Polyglycerinestern ist es nicht möglich, stabile Emulsionen mit gutem Hautgefühl trotz niedriger Viskosität, insbesondere in Verbindung mit einer hohen mikrobiologischen Stabilität, herzustellen.
Aufgabe der Erfindung war es, einen Emulgator bereitzustellen, der mindestens einen Nachteil des Standes der Technik zu überwinden vermag.

Beschreibung der Erfindung

Überraschenderweise wurde gefunden, dass die im Folgenden beschriebenen Polyglycerinester die der Erfindung gestellt Aufgabe zu lösen vermögen.
Gegenstand der vorliegenden Erfindung sind daher Polyglycerinester wie in Anspruch 1 und davon abhängigen Ansprüchen beschrieben.
Ein weiterer Gegenstand der Erfindung ist ein Verfahren zur Herstellung der erfindungsgemäßen Polyglycerinester sowie deren Verwendung als Emulgator oder zur Unterstützung der mikrobiologischen Stabilisierung von Formulierungen und Formulierungen, die diese enthalten.
Ein Vorteil der vorliegenden Erfindung besteht darin, dass der bereitgestellte Emulgator auf Basis des erfindungsgemäßen Polyglycerinesters vollkommen auf nachwachsenden Rohstoffen basiert.
Noch ein Vorteil der vorliegenden Erfindung ist es, dass der bereitgestellte Emulgator auf Basis des erfindungsgemäßen Polyglycerinesters sich zur Formulierung von O/W Emulsionen (Cremes, Lotionen) mit hervorragender Lagerstabilität eignet.
Ein weiterer Vorteil der vorliegenden Erfindung ist es, dass der bereitgestellte Emulgator auf Basis des erfindungsgemäßen Polyglycerinesters sich zur Formulierung PEG-freier Emulsionen, insbesondere dünnflüssiger PEG-freier Emulsionen eignet.
Ein weiterer Vorteil der vorliegenden Erfindung ist es, dass der bereitgestellte Emulgator auf Basis des erfindungsgemäßen Polyglycerinesters sich zur Formulierung von Antitranspirant- und/oder Deo-Formulierungen eignet. Besonders vorteilhaft ist hierbei, dass auch Deosysteme ohne Aluminiumsalze mit hergestellt werden können. Weiterhin besonders vorteilhaft ist, dass die Antitranspirant- und/oder Deo-Formulierungen PEG-frei sein können.
Emulsionen und Formulierungen enthaltend solchen Emulgator auf Basis des erfindungsgemäßen Polyglycerinesters weisen überdies ein gutes Hautgefühl auf.
Der Emulgator auf Basis des erfindungsgemäßen Polyglycerinesters kann Emulsionen gegenüber emulsionsbelastenden Inhaltsstoffen stabilisieren. Hier sind beispielsweise Sonnenschutzformulierungen enthaltend UV-Filter, elektrolythaltige Formulierungen und Formulierungen mit kosmetischen Wirkstoffen, sowie insbesondere pigmenthaltige Emulsionen genannt.

Vorteilhafterweise benötigen Emulsionen und Formulierungen enthaltend solchen Emulgator auf Basis des erfindungsgemäßen Polyglycerinesters nicht zwingend parabenhaltigen Konservierungsmittel, sondern sind auch unter Verwendung anderer Konservierungsmittel mikrobiell stabilisierbar.

Typische Konservierungsmittel sind in Anhang VI der EU-Kosmetikverordnung (76/768/EW) aufgeführte Stoffe wie z.B. Phenoxyethanol, Methylisothiazolinone und Benzoesäure.

In diesem Zusammenhang ist ein weiterer Vorteil der vorliegenden Erfindung, dass die erfindungsgemäßen Polyglycerinester die Wirkung der Konservierungsmittel verstärken können.

Ein weiterer Vorteil der vorliegenden Erfindung ist es, dass der bereitgestellte Emulgator sich zur Formulierung von Emulsionen ohne polyacrylat-basierte Verdicker eignet.

Ein weiterer Vorteil der vorliegenden Erfindung ist es, dass der bereitgestellte Emulgator aufgrund seiner Konsistenz gut handhabbar ist.

Ein weiterer Vorteil der vorliegenden Erfindung ist es, dass der bereitgestellte Emulgator in Formulierungen ein leichtes Hautgefühl erzeugt.

Ein weiterer Vorteil der vorliegenden Erfindung ist es, dass die Verwendung des bereitgestellten Emulgators den Formulierungen feuchtigkeitsspendende Eigenschaften verleiht.

Die Zusammensetzung aus natürlichen Rohstoffen sowie die Fähigkeit des Emulgators auf Basis des erfindungsgemäßen Polyglycerinesters, Emulsionen auch ohne polyacrylat-basierte Verdicker zu stabilisieren, ermöglicht die Herstellung natürlicher Emulsionen entsprechend den Kriterien von Zertifizierungsorganisationen für Naturkosmetik, wie bspw. Natrue, Ecocert oder Cosmos. Das leichte Hautgefühl sowie die feuchtigkeitsspenden Eigenschaften der erfindungsgemäßen Polyglycerinemulgatoren empfehlen die Verwendung dieser in Seren, Feuchtigkeitslotionen, Anti-Aging-Formulierungen oder Blemish Balm Cremes (getönte Wirkstoffformulierung).

Ein weiterer Vorteil der vorliegenden Erfindung ist es, dass die Verwendung des bereitgestellten Emulgators den Formulierungen eine gute Verträglichkeit mit UV Filtern verleiht.

Ein weiterer Vorteil der vorliegenden Erfindung ist es, dass die Verwendung des bereitgestellten Emulgators den Formulierungen eine gute Wirksamkeit bezüglich eingearbeiteter Konservierungsmittel, wie z.B. Phenoxyethanol, verleiht.

Ein weiterer Vorteil der vorliegenden Erfindung ist es, dass die Verwendung des bereitgestellten Emulgators den Formulierungen eine gute Emulsionsstabilität auch in Anwesenheit der eingearbeiteten Konservierungsmitteln verleiht. Besonders vorteilhaft ist hierbei die gute Stabilität auch bei niedrigen pH-Werten, welche erforderlich sind, um eine mikrobiologische Stabilität der kosmetischen Emulsionen unter Verwendung natürlicher Konservierungsmittel wie bspw. Benzoe- oder Sorbinsäure zu erzielen.

Beansprucht wird daher ein Polyglycerinester, der nach seiner vollständiger Hydrolyse im Mittel (Zahlenmittel) pro Mol Polyglycerinester von 1,1 bis 4 Mol, bevorzugt von 1,2 bis 3 Mol, besonders bevorzugt 1,3 bis 2,7 Mol, mindestens einer Carbonsäure mit 8 bis 24 Kohlenstoffatomen freisetzt,

dadurch gekennzeichnet, dass nach vollständiger Hydrolyse des Polyglycerinesters ein Polyglycerin erhalten wird, in dem das Massenverhältnis von Glycerin zu Diglycerin größer 1,5, bevorzugt größer 2, besonders bevorzugt größer 3, ist, und dass nach seiner vollständigen Hydrolyse im Mittel (Zahlenmittel) pro Mol Polyglycerinester von 0,1 bis 1,5 Mol, bevorzugt von 0,2 bis 1,0 Mol, besonders bevorzugt von 0,3 bis 0,7 Mol, mindestens einer ersten Carbonsäure mit 14 bis 24, bevorzugt 16 bis 20, insbesondere 16 bis 18, Kohlenstoffatomen und von 0,5 bis 3,9 Mol, bevorzugt von 0,8 bis 2,5 Mol, besonders bevorzugt von 1,0 bis 2,0 Mol, mindestens einer zweiten Carbonsäure mit 6 bis 12, bevorzugt mit 8 bis 10, besonders bevorzugt von 8, Kohlenstoffatomen, freigesetzt werden.

Bevorzugt ist das vorgenannte Massenverhältnis von Glycerin zu Diglycerin kleiner als 15.

Unter dem Begriff "Polyglycerin" im Sinne der vorliegenden Erfindung ist ein Polyglycerin zu verstehen, welches Glycerin enthält. Somit ist zur Berechnung von Mengen, Massen und dergleichen der Glycerinanteil mit zu berücksichtigen. Polyglycerine im Sinne der vorliegenden Erfindung sind somit Mischungen enthaltend Glycerin und mindestens ein Glycerin-Oligomer. Unter Glycerin-Oligomeren sind jeweils alle entsprechenden Strukturen, z.B. also lineare und zyklische Verbindungen zu verstehen.

Analoges gilt für den Begriff "Polyglycerinester" im Zusammenhang mit der vorliegenden Erfindung.

Das angegebene Zahlenmittel der Säurereste bezieht sich bei mehr als einer der ersten und/oder zweiten Carbonsäure jeweils auf die akkumulierte Summe aller ersten und/oder zweiten Säurereste.

Alle angegebenen Prozent (%) sind, wenn nicht anders angegeben, Massenprozent. Der Massenanteil von Glycerin, Diglycerin, Triglycerin sowie der Fettsäuren lässt sich im Rahmen der vorliegenden Erfindung durch zwei GC-Methoden bestimmen; diese Methoden beinhalten die alkalische Hydrolyse des erfindungsgemäßen Polyglycerinesters, Trennung des Polyglycerins von den entstandenen Säuren und Analyse der Fettsäuren sowie der Glycerin-Oligomere (lineare und zyklische).

Hierzu werden 0,5 g des erfindungsgemäßen Polyglycerinesters in 25 ml einer ethanolischen 0,5 M KOH Lösung unter Rückfluss für 4 Stunden gekocht. Dann werden 10 ml Wasser zugegeben und der pH mit Schwefelsäure auf pH 2-3 eingestellt. Die entstandenen Carbonsäuren werden durch dreimalige Extraktion mit jeweils einem Volumen (30 ml) Petrolether abgetrennt.

Fettsäureanalyse :

Die vereinigten Extrakte werden durch Evaporation auf ca. 1 ml eingeengt. Geeignete Bestimmungsmethoden zur Ermittlung der Fettsäureverteilung sind insbesondere solche gemäß DGF C VI 11a, DGF C-VI 10 a und GAT - Ringtest 7/99.

Ein 0,5 ml Aliquot des wie oben beschriebenen erhaltenen Petroletherextraktes wird in einem Gefäß mit 1 ml einer Mischung von Acetylchlorid: Methanol (1:4) und 1 ml Chloroform versetzt und mittels GC analysiert. Dieses wird in einem Gaschromatograph, welcher mit einem split/splitless Injektor, einer Kapillarsäule und einem Flammenionisationdetektor ausgestattet ist, unter folgenden Bedingungen durchgeführt:

| | |
|---|---|
| Injektor: | 290 °C, Split 30 ml |
| Injektionsvolumen: | 1 $\mu$l |
| Säule: | 30 m *0,32 mm HP1 0,25 $\mu$m |
| Trägergas: | Helium, constant flow, 2 mL/min |
| Temperaturprogramm: | 80 °C - 300 °C mit 4 °C/min, dann 10 Minuten bei 300 °C konditionieren. |
| Detektor: | FID bei 320 °C |
| | Wasserstoff 35 ml/min |
| | Luft 240 ml/min |
| | Make Up Gas 12 ml/min |

Die Carbonsäuren werden als ihre Methylester nach ihrer Kohlenstoffkettenlänge getrennt und ihr Massenanteil nach einer Methode des internen Standards bestimmt. Hierzu wird das GC System durch Vermessen von Fettsäuremethylester-Mischungen der zu untersuchenden Fettsäuren mit bekannter Zusammensetzung kalibriert.

Mit diesem Verfahren wird die Gesamtmasse und die Massenanteile an Carbonsäure(n) erhalten, die über die Verwendung der jeweiligen Molekulargewichte eine Bestimmung der Stoffemenge(n) erlaubt. Aus der Gesamtmasse an Carbonsäure(n) lässt sich außerdem durch Subtraktion die z.B. in 0.5 g Polyglycerinester enthaltene Masse an Polyglycerin bestimmen.

Mittels des Molekulargewichts des Polyglycerins lässt sich daraus die Stoffmenge des Polyglycerins bestimmen.

| | | |
|---|---|---|
| $M_p = 74 \cdot N + 18$ | mit | $M_p$ = Molekulargewicht des Polyglycerins [g/mol] <br> N = Polymerisationsgrad des Polyglycerins (zur Bestimmung des Polymerisationsgrads siehe weiter unten). |
| $n_p = \dfrac{m_p}{M_p}$ | mit | $n_p$ = Stoffmenge des Polyglycerins [mol] in 1 g Polyglycerinester <br> $m_p$ = Masse Polyglycerin in 1 g Polyglycerinester [g] <br> $M_p$ = Molekulargewicht des Polyglycerins [g/mol] |

Zusammen lassen sich aus diesen Werten die Molverhältnisse von Polyglycerin zu Carbonsäuren bestimmen.

Analyse von Glycerin, Diglycerin und Triglycerin:

Der mit Petrolether extrahierte Rückstand wird mit Bariumhydroxid auf pH 7 bis 8 eingestellt. Das ausgefallene Bariumsulfat wird durch Zentrifugation abgetrennt. Der Überstand wird abgenommen und der Rückstand wird dreimal mit 20 ml Ethanol extrahiert.

Die vereinigten Überstände werden für 30 min bei 80 °C und 50 mbar eingeengt und getrocknet.

Für die Analyse Glycerin, Diglycerin und Triglycerin mittels GC wird der Rückstand in 2 ml Pyridin:Chloroform (4:1) gelöst. 0,5 ml dieser Lösung werden mit 1 ml MSTFA [N-Methyl-N-(trimethylsilyl) trifluoroacetamide] versetzt. Die Alkohole werden durch Reaktion bei 80°C (30 Minuten) quantitativ in ihre Trimethylsilyether überführt und anschließend mittels GC/FID untersucht.

Dieses wird in einem Gaschromatograph, welcher mit einem split/splitless Injektor, einer Kapillarsäule und einem Flammenionisationdetektor ausgestattet ist, unter folgenden Bedingungen durchgeführt:

| | |
|---|---|
| Injektor: | 290 °C, Split 30 ml |
| Injektionsvolumen: | 1 $\mu$l |

(fortgesetzt)

| | |
|---|---|
| Säule: | 30 m *0,32 mm HP1 0,25 μm |
| Trägergas: | Helium, constant flow, 2 mL/min |
| Temperaturprogramm: | 80 °C - 300 °C mit 4 °C/min, dann 10 Minuten bei 300 °C konditionieren. |
| Detektor: | FID bei 310 °C |
| | Wasserstoff 35 ml/min |
| | Luft 240 ml/min |
| | Make Up Gas 12 ml/min |

Glycerin, Diglycerin und Triglycerin werden aufgetrennt und ihr Massenanteil nach einer Methode des internen Standards bestimmt. Hierzu wird das GC System durch Vermessen von Mischungen der zu untersuchenden Glycerine und des internen Standards mit bekannter Zusammensetzung kalibriert.

Aus den Massenanteilen lässt sich das Massenverhältnis von Glycerin zu Diglycerin bestimmen sowie durch Subtraktion von 100% auch der Gehalt an Polyglycerinen mit einem Polymerisationsgrad von 2 und größer (100% minus Massenanteil des Glycerins), der Gehalt an Polyglycerinen mit einem Polymerisationsgrad von 3 und größer (100% minus Massenanteile des Glycerins und der Diglycerine) und der Gehalt an Polyglycerinen mit einem Polymerisationsgrad von 4 und größer (100% minus Massenanteile des Glycerins, der Diglycerine und der Triglycerine).

Sollte in einem betrachteten Polyglycerin keine nachweisbare Menge an Diglycerin, jedoch Glycerin enthalten sein, so entspricht dies einem Massenverhältnis von Glycerin zu Diglycerin größer 3.

Es ist erfindungsgemäß bevorzugt, dass das nach vollständiger Hydrolyse des erfindungsgemäßen Polyglycerinesters erhaltene Polyglycerin einen mittleren Polymerisationsgrad von 1,5 bis 10, bevorzugt von 1,7 bis 6, besonders bevorzugt von 2 bis 3,5, aufweist.

Der mittlere Polymerisationsgrad des Polyglycerins $N$ wird über dessen Hydroxylzahl (OHV, in mg KOH/g) gemäß folgender Formel berechnet:

$$N = \frac{(112200 - 18 \cdot OHV)}{(74 \cdot OHV - 56100)}$$

Geeignete Bestimmungsmethoden zur Ermittlung der Hydroxylzahl sind insbesondere solche gemäß DGF C-V 17 a (53), Ph. Eur. 2.5.3 Method A und DIN 53240.

Es ist erfindungsgemäß bevorzugt, dass das nach vollständiger Hydrolyse des erfindungsgemäßen Polyglycerinesters erhaltene Polyglycerin mindestens 50 Gew.-%, bevorzugt mindestens 60 Gew.-%, Polyglycerine mit einem Polymerisationsgrad von 2 und größer enthält, wobei sich die Gewichtsprozente auf den gesamten Gehalt an Polyglycerin beziehen.

Es ist erfindungsgemäß bevorzugt, dass das nach vollständiger Hydrolyse des erfindungsgemäßen Polyglycerinesters erhaltene Polyglycerin mindestens 40 Gew.-%, bevorzugt mindestens 50 Gew.-%, Polyglycerine mit einem Polymerisationsgrad von 3 und größer enthält, wobei sich die Gewichtsprozente auf den gesamten Gehalt an Polyglycerin beziehen.

Es ist erfindungsgemäß bevorzugt, dass das nach vollständiger Hydrolyse des erfindungsgemäßen Polyglycerinesters erhaltene Polyglycerin mindestens 30 Gew.-%, bevorzugt mindestens 40 Gew.-%, Polyglycerine mit einem Polymerisationsgrad von 4 und größer enthält, wobei sich die Gewichtsprozente auf den gesamten Gehalt an Polyglycerin beziehen.

Es ist erfindungsgemäß besonders bevorzugt, dass das nach vollständiger Hydrolyse des erfindungsgemäßen Polyglycerinesters erhaltene Polyglycerin mindestens 50 Gew.-%, bevorzugt mindestens 60 Gew.-%, Polyglycerine mit einem Polymerisationsgrad von 2 und größer,

mindestens 40 Gew.-%, bevorzugt mindestens 50 Gew.-%, Polyglycerine mit einem Polymerisationsgrad von 3 und größer und

mindestens 30 Gew.-%, bevorzugt mindestens 40 Gew.-%, Polyglycerine mit einem Polymerisationsgrad von 4 und größer enthält,

wobei sich die Gewichtsprozente auf den gesamten Gehalt an Polyglycerin beziehen.

Vorteilhafte Polyglycerinester gemäß der vorliegenden Erfindung sind dadurch gekennzeichnet, dass das molare Verhältnis der nach vollständiger Hydrolyse des Polyglycerinesters erhaltenen ersten Carbonsäuren zur zweiten Carbonsäure zwischen 1 zu 1,5 bis 1 zu 5,0, bevorzugt zwischen 1 zu 2,0 bis 1 zu 4,0, besonders bevorzugt zwischen 1 zu 2,5 und 1 zu 3,0, liegt.

Zur Bestimmung der Molverhältnisse lässt sich als Methode die oben beschriebene Methode einsetzen.

Es ist erfindungsgemäß bevorzugt, dass die erste und zweite Carbonsäure des Polyglycerinesters ausgewählt ist aus Fettsäuren, solche sind insbesondere ausgewählt aus linearen, gesättigten, unsubstituierten Carbonsäuren.

Als Fettsäuren lassen sich sowohl für die erste als auch die zweite Carbonsäure Mischungen von Fettsäuren einsetzen, wobei es für die erste Carbonsäure bevorzugt ist, dass es sich in diesem Zusammenhang um eine Mischung handelt. Dies liegt in der Natur des Herstellungsverfahrens begründet, in dem bevorzugt technische Mischungen von Fettsäuren eingesetzt werden.

Ein erfindungsgemäß bevorzugter Polyglycerinester ist dadurch gekennzeichnet, dass er als erste Carbonsäure solche enthält ausgewählt aus Stearinsäure und Palmitinsäure, sowie Mischungen hieraus. Bevorzugte Mischungen können im Gewichtsverhältnis Stearinsäure zu Palmitinsäure von 100:0,01 zu 0,01:100 breit variiert werden. Bevorzugt ist dabei ein C18:C16-Gewichtsverhältnis von 30:70 bis 95:5, besonders ein Verhältnis von 45:55 bis 90:10. Bevorzugte zweite Carbonsäure ist Caprylsäure.

Eine besonders bevorzugt Ausführungsform des erfindungsgemäßen Polyglycerinesters ist dadurch gekennzeichnet, dass die erste Carbonsäure eine Mischung von Stearinsäure und Palmitinsäure ist, die bevorzugt ein Gewichtsverhältnis in einem Bereich von 0,8 zu 1 bis 1,2 zu 1 aufweist, und die zweite Carbonsäure Caprylsäure ist.

Die Polyglycerinester der vorliegenden Erfindung lassen sich durch klassische Veresterungsverfahren herstellen, bevorzugt mit dem im Folgenden beschriebenen erfindungsgemäßen Verfahren.

Ein weiterer Gegenstand der vorliegenden Erfindung ist ein Verfahren zur Herstellung eines Polyglycerinesters umfassend die Verfahrensschritte

A) Bereitstellen eines Polyglycerins, in dem das Massenverhältnis von Glycerin zu Diglycerin größer 1,5, bevorzugt größer 2, besonders bevorzugt größer 3 ist,

B) Verestern mindestens eines Teils des Polyglycerins mit mindestens einer Carbonsäure mit 8 bis 24 Kohlenstoffatomen,

wobei das molare Verhältnis der in Verfahrensschritt B) eingesetzten Carbonsäure zu in Verfahrensschritt A) eingesetztem Polyglycerin von 1,1 zu 1 bis 4 zu 1, bevorzugt von 1,2 zu 1 bis 3 zu 1, besonders bevorzugt 1,3 zu 1 bis 2,7 zu 1, beträgt, dadurch gekennzeichnet, dass in Verfahrensschritt B) mindestens eine erste Carbonsäure mit 14 bis 24 Kohlenstoffatomen und mindestens eine zweite Carbonsäure mit 6 bis 12 Kohlenstoffatomen eingesetzt wird,

wobei das molare Verhältnis der in Verfahrensschritt B) eingesetzten ersten Carbonsäure zu in Verfahrensschritt A) eingesetztem Polyglycerin von 0,1 zu 1 bis 1,5 zu 1, bevorzugt von 0,2 zu 1 bis 1,0 zu 1, besonders bevorzugt von 0,3 zu 1 bis 0,7 zu 1, und das molare Verhältnis der in Verfahrensschritt B) eingesetzten zweiten Carbonsäure zu in Verfahrensschritt A) eingesetztem Polyglycerin von 0,5 zu 1 bis 3,9 zu 1, bevorzugt von 0,8 zu 1 bis 2,5 zu 1, besonders bevorzugt von 1,0 zu 1 bis 2,0 zu 1, beträgt.

Das Polyglycerin für Verfahrensschritt A) kann bereitgestellt werden durch verschiedene konventionelle Methoden wie beispielsweise Polymerisation von Glycidol (z.B. basenkatalysiert), Polymerisation von Epichlorhydrin (beispielsweise in Gegenwart äquimolarer Mengen einer Base wie NaOH) oder Polykondensation von Glycerin.

Erfindungsgemäß bevorzugt ist die Bereitstellung des Polyglycerins durch die Kondensation von Glycerin, insbesondere in Gegenwart katalytischer Mengen einer Base, insbesondere NaOH oder KOH. Geeignete Reaktionsbedingungen sind Temperaturen zwischen 220-260 °C und reduzierter Druck in einem Bereich zwischen 20 bis 800 mbar, insbesondere zwischen 50 und 500 mbar, wodurch eine erleichterte Wasserentfernung möglich ist. Entsprechende Verfahren lassen sich Standardlehrwerken der Chemie wie beispielsweise dem Römpp entnehmen.

Es kann vorteilhaft sein, ein konventionell erhaltenes Polyglycerin durch Beimischen von Glycerin auf das erfindungsgemäß benötigte Verhältnis von Glycerin zu Diglycerin zu bringen.

Es ist bevorzugt, wenn das in Verfahrensschritt A) bereitgestellte Polyglycerin einen mittleren Polymerisationsgrad von 1,5 bis 10, bevorzugt von 1,7 bis 6, besonders bevorzugt von 2 bis 3,5, aufweist.

Es ist erfindungsgemäß bevorzugt, wenn das in Verfahrensschritt A) bereitgestellte Polyglycerin

mindestens 50 Gew.-%, bevorzugt mindestens 60 Gew.-%, Polyglycerine mit einem Polymerisationsgrad von 2 und größer enthält, wobei sich die Gewichtsprozente auf den gesamten Gehalt an Polyglycerin beziehen.

Es ist erfindungsgemäß bevorzugt, wenn das in Verfahrensschritt A) bereitgestellte Polyglycerin

mindestens 40 Gew.-%, bevorzugt mindestens 50 Gew.-%, Polyglycerine mit einem Polymerisationsgrad von 3 und größer enthält, wobei sich die Gewichtsprozente auf den gesamten Gehalt an Polyglycerin beziehen.

Es ist erfindungsgemäß bevorzugt, wenn das in Verfahrensschritt A) bereitgestellte Polyglycerin

mindestens 30 Gew.-%, bevorzugt mindestens 40 Gew.-%, Polyglycerine mit einem Polymerisationsgrad von 4 und größer enthält, wobei sich die Gewichtsprozente auf den gesamten Gehalt an Polyglycerin beziehen.

Es ist erfindungsgemäß besonders bevorzugt, wenn das in Verfahrensschritt A) bereitgestellte Polyglycerin

mindestens 50 Gew.-%, bevorzugt mindestens 60 Gew.-%, Polyglycerine mit einem Polymerisationsgrad von 2 und größer,

mindestens 40 Gew.-%, bevorzugt mindestens 50 Gew.-%, Polyglycerine mit einem Polymerisationsgrad von 3 und größer und

mindestens 30 Gew.-%, bevorzugt mindestens 40 Gew.-%, Polyglycerine mit einem Polymerisationsgrad von 4 und größer enthält,

wobei sich die Gewichtsprozente auf den gesamten Gehalt an Polyglycerin beziehen.

In dem erfindungsgemäßen Verfahren werden die Verfahrensschritte B) unter dem Fachmann bekannten Bedingungen für Veresterungsreaktionen, gegebenenfalls in Anwesenheit eines Katalysators, durchgeführt. Insbesondere wird diese Veresterung unter Entfernung von Wasser aus dem Reaktionsgemisch durchgeführt.

Verfahrensschritt B) wird bevorzugt bei 180-260 °C, besonders bevorzugt 210-250°C durchgeführt.

Der Reaktionsverlauf kann beispielsweise über die Säurezahl des Produktes überwacht werden, so dass es in Verfahrensschritt B) bevorzugt ist, diesen so lange durchzuführen, bis die gewünschte Säurezahl erreicht wird.

Bevorzugt werden in Verfahrensschritt B) die erste und zweite Carbonsäure in einem molaren Verhältnis zwischen 1 zu 1,5 bis 1 zu 5,0, bevorzugt zwischen 1 zu 2,0 bis 1 zu 4,0, besonders bevorzugt zwischen 1 zu 2,5 und 1 zu 3,0, eingesetzt. Die in dem erfindungsgemäßen Verfahren in Verfahrensschritt B) eingesetzten ersten und zweiten Carbonsäuren sind bevorzugt solche, die oben als bevorzugte in erfindungsgemäßen Polyglycerinestern genannt wurden.

Insbesondere werden in diesem Zusammenhang als die erste Carbonsäure eine Mischung von Stearinsäure und Palmitinsäure, die bevorzugt ein Gewichtsverhältnis von Stearinsäure zu Palmitinsäure in einem Bereich von 0,8 zu 1 bis 1,2 zu 1 aufweist, und als die zweite Carbonsäure Caprylsäure eingesetzt.

Erfindungsgemäße Polyglycerinester und Polyglycerinester erhältlich bzw. erhalten nach dem erfindungsgemäßen Verfahren eignen sich hervorragend zur Verwendung als leistungsstarker O/W Emulgator, der ausschließlich auf nachwachsenden Rohstoffen basiert und eine große Formulierungsflexibilität aufweist, insbesondere in kosmetischen Formulierungen.

Somit sind Emulgatoren enthaltend erfindungsgemäße Polyglycerinester oder Polyglycerinester erhältlich bzw. erhalten nach dem erfindungsgemäßen Verfahren Gegenstand der vorliegenden Erfindung. Als Emulgator ist im Rahmen dieser Erfindung ein Emulgator zu verstehen, der mindestens aus einem erfindungsgemäßen Polyglycerinester oder Polyglycerinester erhältlich bzw. erhalten nach dem erfindungsgemäßen Verfahren und gegebenenfalls mindestens einem Co-Emulgator besteht, wobei die Anwesenheit eines Co-Emulgators bevorzugt ist.

Als Co-Emulgator können vorteilhaft folgende Co-Emulgatoren eingesetzt werden: Polyglyceryl-3 Dicitrate/Stearate, Polyglyceryl-3 Methylglucose Distearate, Polyglyceryl-10 Stearate, Polyglyceryl-6 Distearate, Methylglucose Sesquistearate, Sodium Stearoyl Glutamate, Sodium Cetearyl Sulfate, Potassium Cetyl Phosphate, Glyceryl Stearate Citrate, Cetearyl Glucoside, Glyceryl Stearate, Glyceryl Stearate SE, Cetearyl Alcohol und Stearic Acid.

Ebenso eignen sich erfindungsgemäße Polyglycerinester und Polyglycerinester erhältlich bzw. erhalten nach dem erfindungsgemäßen Verfahren hervorragend zur Verwendung zur Herstellung von kosmetischen oder pharmazeutischen Formulierungen, insbesondere zur Herstellung kosmetischer Cremes und Lotionen. Unter Cremes und Lotionen wird in diesem Zusammenhang kosmetische O/W Emulsionen mit streichfähig-pastöser bzw. fließfähiger Konsistenz verstanden. Allgemein können die erfindungsgemäßen Polyglycerinester beispielsweise in Pflegecremes und -lotionen für Gesicht, Körper und Hände, in Sonnenschutzemulsionen, in Seren, in BB Cremes, in Deodorants, in Schminken, in Aersolen, Roll-ons, Pumpsprays, Stiften z.B. im AP/Deo-Bereich, in Babypflegeprodukten, in Intimpflege-, Fußpflege-, Haarpflege-, Nagelpflege, Zahnpflege- oder Mundpflegeprodukten sowie in dermatologischen Salben eingesetzt werden.

Somit sind ebenfalls kosmetischen oder pharmazeutischen Formulierungen, insbesondere O/W Formulierungen, enthaltend erfindungsgemäße Polyglycerinester oder Polyglycerinester erhältlich bzw. erhalten nach dem erfindungsgemäßen Verfahren Gegenstand der vorliegenden Erfindung. Erfindungsgemäß bevorzugte Formulierungen stellen Sonnenschutzpräparate, elektrolythaltige Emulsionen, Formulierungen mit kosmetischen Wirkstoffen und O/W Make-Up Formulierungen dar.

Erfindungsgemäß bevorzugte Formulierungen, enthalten den erfindungsgemäßen Polyglycerinester oder Polyglycerinester erhältlich bzw. erhalten nach dem erfindungsgemäßen Verfahren in Mengen von 0,01 bis 10 Gew.-%, bevorzugt 0,05 bis 8 Gew.-% und besonders bevorzugt 0,1 bis 7 Gew.-% bezogen auf die Gesamtformulierung.

Insbesondere kosmetische oder pharmazeutische Formulierungen sind bevorzugt, die im Wesentlichen frei von alkoxylierten Verbindungen sind. Unter dem Begriff "im Wesentlichen frei von alkoxylierten Verbindungen" im Zusammenhang mit der vorliegenden Erfindung ist zu verstehen, dass die Formulierungen keine nennenswerten Mengen an alkoxylierten Verbindungen aufweisen, die eine oberflächenaktive Wirkung ausüben. Insbesondere ist hierunter zu verstehen, dass diese Verbindungen in Mengen von kleiner 1 Gew.-%, bevorzugt von kleiner 0,1 Gew.-%, besonders bevorzugt von kleiner 0,01 Gew.-% bezogen auf die Gesamtformulierung, insbesondere keine nachweisbaren Mengen, enthalten sind.

Die erfindungsgemäßen Formulierungen können z.B. mindestens eine zusätzliche Komponente enthalten, ausgewählt aus der Gruppe der

Emollients,
Co-Emulgatoren,

Verdicker/Viskositätsregler/Stabilisatoren,
Antioxidantien,
Hydrotrope (oder Polyole),
Fest- und Füllstoffe,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
Pigmente
kosmetische Wirkstoffe,
Pflegeadditive,
Überfettungsmittel,
Lösungsmittel,
UV-Filter,
Elektrolyte,
Multifunktionelle Additive,
feuchtigkeitsspendende Stoffe.

Substanzen, die als beispielhafte Vertreter der einzelnen Gruppen eingesetzt werden können, sind dem Fachmann bekannt und können beispielsweise der deutschen Anmeldung DE 102008001788.4 entnommen werden. Diese Patentanmeldung wird hiermit als Referenz eingeführt und gilt somit als Teil der Offenbarung.
Bezüglich weiterer fakultativer Komponenten sowie deren eingesetzten Mengen dieser Komponenten wird ausdrücklich auf die dem Fachmann bekannten einschlägigen Handbücher, z. B. K. Schrader, "Grundlagen und Rezepturen der Kosmetika", 2. Auflage, Seite 329 bis 341, Hüthig Buch Verlag Heidelberg, verwiesen.
Die Mengen der jeweiligen Zusätze richten sich nach der beabsichtigten Verwendung.
Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.
Da die erfindungsgemäßen Polyglycerinester Pigmente oder Festkörper überaus stabil in Emulsionspräparaten halten können, sind Fest- und Füllstoffe, insbesondere Partikel und Additive, die zur Erzielung eines spezifischen Hautgefühls eingesetzt werden, wie z.B. Siliconelastomere, PMMA-Partikel, PE-Partikel, PS-Partikel, Nylon-Partikel, Bornitrid, Stärke, Cellulose, Mica und Talkum, eine bevorzugte zusätzliche Komponente.
Neben den genannten Partikeln können auch unlösliche UV-Filter in Form von Pigmenten, nämlich feindisperse Metalloxide bzw. Salze vorteilhaft enthalten sein, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, z.B. zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Eine relativ neue Klasse von Lichtschutzfiltern sind micronisierte organische Pigmente, wie beispielsweise 2,2'-Methylene-bis-{6-(2H-benzotriazole-2-yl)-4-(1, 1, 3, 3-tetramethylbutyl)-phenol} mit einer Partikelgröße von < 200 nm, das z.B. als 50 %ige wässrige Dispersion erhältlich ist. Ebenso ganz besonders vorteilhaft geeignet ist die vorliegende Erfindung zur Stabilisierung kosmetischen Farbpigmente enthaltender Formulierungen.
Daher enthält eine bevorzugte kosmetische oder pharmazeutische Formulierung als zusätzliche Komponente eine Substanz ausgewählt aus der Gruppe der Substanzen der kosmetischen Farbpigmente.
Kosmetischen Farbpigmente im Zusammenhang mit der vorliegenden Erfindung sind insbesondere ausgewählt aus der Gruppe bestehend aus Metalloxiden oder Mischungen davon, wobei Eisenoxide besonders bevorzugt sind.
Die Verwendung des Emulgators in Formulierungen mit Elektrolyten oder ionischen Verbindungen ist ebenfalls ein vorteilhaftes Anwendungsgebiet. Sogenannte multifunktionelle Additive, welche unter anderem feuchtigkeitsspendende, konservierende, viskositätsregulierende, emulgierende, stabilisierende Wirkung haben können, sind mit dem erfindungsgemäßen Emulgator ebenfalls sehr gut in O/W Emulsionen, besonders in Lotionen, zu formulieren, weshalb Vertreter dieser Stoffgruppe ebenfalls eine bevorzugte zusätzliche Komponente darstellen.
Da die erfindungsgemäßen Polyglycerinester in der Lage sind, Emulsionen auch ohne die Verwendung von polyacrylatbasierten Verdickern zu stabilisieren, sind für sie PEG- und oder silikon- und oder polyacrylat- und oder paraben-freie Formulierungen auf Basis natürlicher Inhaltsstoffe ein besonders bevorzugtes Anwendungsgebiet.

Aufgrund seiner feuchtigkeitsspendenden Eigenschaften, seines leichten Hautgefühls und der guten Stabilisierung kosmetischer Wirkstoffe eignen sich erfindungsgemäße Polyglycerinesters beispielsweise bevorzugt für die Verwendung in kosmetischen Emulsionen zur Steigerung der Hautfeuchtigkeit, Verbesserung der Hautstruktur, Verringerung altersbedingter Hautveränderungen, Nivellierungs des Hauttaints, Förderung des Hautstoffwechsels oder Neutralisierung schädlicher Stoffwechselbestandteile in der Haut. Daher sind feuchtigkeitsspende Stoffe und Stoffe mit biologischer Wirksamkeit, wie Glycerin, Harnstoff, Peptide, bevorzugte zusätzliche Komponenten in bevorzugten erfindungsgemäßen Formulierungen. Aufgrund der Toleranz der erfindungsgemäßen Emulgatoren gegenüber verschiedenartigen Konservierungsmitteln sind neben parabenhaltigen Konservierungsmitteln auch parabenfreie Konservierungsmittel wie Phenoxyethanol, Ethanol oder Methylisothiazolinon gut einsetzbar. Zur Herstellung von Naturkosmetik sind als Konservierungsmittel vor allem natürliche Säuren wie Caprylsäure, Caprinsäure, Anissäure, Sorbitansäure, Lävulinsäure, Sorbinsäure und Benzoesäure oder Stoffe wie Caprylyl Glycol bevorzugte Konservierungsmittel, die als zusätzlicher Formulierungsbestandteil eingesetzt werden.

Bei bevorzugten erfindungsgemäßen Formulierungen handelt es sich um Antiperspirant und/oder Deodorant-Formulierungen. Antiperspirant Formulierungen sind dadurch gekennzeichnet, dass sie augenfällig hergerichtet wurden, um Schweißbildung vorzubeugen. Deodorant-Formulierungen sind dadurch gekennzeichnet, dass sie augenfällig hergerichtet wurden, um Geruchsbildung vorzubeugen. Insbesondere enthalten Antiperspirant und/oder Deodorant-Formulierungen Wirkstoffe ausgewählt aus der Gruppe Aluminiusalze.

Bevorzugte erfindungsgemäße Formulierungen sind dadurch gekennzeichnet, dass sie einen pH-Wert von 3,0 bis 7,0, bevorzugt von 3,5 bis 6, aufweisen.

Der "pH-Wert" im Zusammenhang mit der vorliegenden Erfindung ist definiert als der Wert, welcher bei 25 °C nach fünf Minuten Rühren gemessen wird mit einer kalibrierten pH-Elektrode gemäß ISO 4319 (1977).

Ein weiterer Gegenstand der vorliegenden Erfindung ist die Verwendung der erfindungsgemäßen Polyglycerinester zur Unterstützung der mikrobiologischen Stabilisierung von Formulierungen.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Folgende Abbildungen sind Bestandteil der Beispiele:

Beispiele:

*Synthesebeispiel 1), erfindungsgemäß*

Synthesebeispiel 1a)

Ein Gemisch aus Glycerin (2102 g, 22,8 mol) und 45%iger wässriger Kalilauge (24,2 g) wurde bei 400 mbar innerhalb von 1 Stunde auf 240 °C erhitzt und das entstehende Wasser kontinuierlich abdestilliert. Sobald das Reaktionsgemisch einen Brechungsindex von ≥1.4900 (typischerweise nach 20-21 h bei 240 °C) erreicht hatte, wurde der Druck langsam auf 50 mbar gesenkt und weiter Wasser sowie überschüssiges Glycerin bei 240 °C abdestilliert, bis das verbleibende Gemisch eine Hydroxylzahl von 880 mg KOH/g aufwies.

Ein Gemisch aus dem so erhaltenen Polyglycerin (554,8 g, 0,75 mol) und Stearinsäure und Palmitinsäure im Verhältnis 1:1 (477,1 g, 1,77 mol) wurde innerhalb von 3 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von ≤10 erreicht war.

Synthesebeispiel 1b)

Ein Gemisch aus Glycerin (267,3 g, 2,90 mol) und Caprylsäure (837,0 g, 5,79 mol) wurde innerhalb von 3 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von ≤10 erreicht war.

Synthesebeispiel 1c)

Ein Gemisch eines Polyglycerinesters erhalten wie beschrieben in Beispiel 1a) (50 g) und eines Polyglycerinesters erhalten wie beschrieben in Beispiel 1b) (50 g) wurde innerhalb von 3 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von ≤1.0 erreicht war.

Der so erhaltene Polyglycerinester setzt nach seiner vollständigen Hydrolyse pro Mol Polyglycerinester

ca. 0,4 mol Stearinsäure und Palmitinsäure im Verhältnis 1:1 und

ca. 1,4 mol Caprylsäure frei und

ist dadurch gekennzeichnet, dass das nach vollständiger Hydrolyse des Polyglycerinesters freigesetzte Polyglycerin ein Massenverhältnis von Glycerin zu Diglycerin von ca. 4 aufweist. Das nach vollständiger Hydrolyse erhaltene Polyglycerin weist einen Polymerisationsgrad von ca. 3 auf und einen Gehalt an Polyglycerinen mit einem Polymerisationsgrad von 2 und größer von größer 60%, einen Gehalt an Polyglycerinen mit einem Polymerisationsgrad von 3 und größer von größer 50% und einen Gehalt an Polyglycerinen mit einem Polymerisationsgrad von 4 und größer von größer 40%.

*Synthesebeispiel 2), erfindungsgemäß*

Synthesebeispiel 2a)

Ein Gemisch aus Glycerin (2102 g, 22,8 mol) und 45%iger wässriger Kalilauge (24,2 g) wurde bei 400 mbar innerhalb von 1 Stunde auf 240 °C erhitzt und das entstehende Wasser kontinuierlich abdestilliert. Sobald das Reaktionsgemisch einen Brechungsindex von ≥1.4900 (typischerweise nach 20-21 h bei 240 °C) erreicht hatte, wurde der Druck langsam auf 50 mbar gesenkt und weiter Wasser sowie überschüssiges Glycerin bei 240 °C abdestilliert, bis das verbleibende Gemisch eine Hydroxylzahl von 880 mg KOH/g aufwies.

Ein Gemisch aus dem so erhaltenen Polyglycerin (554,8 g, 0,75 mol) und Stearinsäure und Palmitinsäure im Verhältnis 1:1 (477,1 g, 1,77 mol) wurde innerhalb von 3 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von ≤10 erreicht war.

Synthesebeispiel 2b)

Ein Gemisch aus Glycerin (267,3 g, 2,90 mol) und Caprylsäure (767,9 g, 5,31 mol) wurde innerhalb von 3 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von ≤10 erreicht war.

Synthesebeispiel 2c)

Ein Gemisch eines Polyglycerinesters erhalten wie beschrieben in Beispiel 2a) (50 g) und eines Polyglycerinesters erhalten wie beschrieben in Beispiel 2b) (50 g) wurde innerhalb von 3 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von ≤1.0 erreicht war.

Der so erhaltene Polyglycerinester setzt nach seiner vollständigen Hydrolyse pro Mol Polyglycerinester

ca. 0,4 mol Stearinsäure und Palmitinsäure im Verhältnis 1:1 und

ca. 1,3 mol Caprylsäure frei und

ist dadurch gekennzeichnet, dass das nach vollständiger Hydrolyse des Polyglycerinesters freigesetzte Polyglycerin ein Massenverhältnis von Glycerin zu Diglycerin von ca. 4 aufweist. Das nach vollständiger Hydrolyse erhaltene Polyglycerin weist einen Polymerisationsgrad von ca. 3 auf und einen Gehalt an Polyglycerinen mit einem Polymerisationsgrad von 2 und größer von größer 60%, einen Gehalt an Polyglycerinen mit einem Polymerisationsgrad von 3 und größer von größer 50% und einen Gehalt an Polyglycerinen mit einem Polymerisationsgrad von 4 und größer von größer 40%.

*Synthesebeispiel 3), erfindungsgemäß*

Ein Gemisch aus Glycerin (2102 g, 22,8 mol) und 45%iger wässriger Kalilauge (24,2 g) wurde bei 400 mbar innerhalb von 1 Stunde auf 240 °C erhitzt und das entstehende Wasser kontinuierlich abdestilliert. Sobald das Reaktionsgemisch einen Brechungsindex von ≥1.4900 (typischerweise nach 20-21 h bei 240 °C) erreicht hatte, wurde der Druck langsam auf 50 mbar gesenkt und weiter Wasser sowie überschüssiges Glycerin bei 240 °C abdestilliert, bis das verbleibende Gemisch eine Hydroxylzahl von 880 mg KOH/g aufwies.

Ein Gemisch aus dem so erhaltenen Polyglycerin (147,0 g, 0,20 mol) Glycerin (73,7 g, 0,8 Mol) und Stearinsäure und Palmitinsäure im Verhältnis 1:1 (129,5 g, 0,48 mol) und Caprylsäure (228,5 g, 1,58 mol) wurde innerhalb von 3 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von ≤1.0 erreicht war.

Der so erhaltene Polyglycerinester setzt nach seiner vollständigen Hydrolyse pro Mol Polyglycerinester

ca. 0,5 mol Stearinsäure und Palmitinsäure im Verhältnis 1:1 und

ca. 1,6 mol Caprylsäure frei und

ist dadurch gekennzeichnet, dass das nach vollständiger Hydrolyse des Polyglycerinesters freigesetzte Polyglycerin ein Massenverhältnis von Glycerin zu Diglycerin von ca. 4 aufweist. Das nach vollständiger Hydrolyse erhaltene Polyglycerin weist einen Polymerisationsgrad von ca. 3 auf und einen Gehalt an Polyglycerinen mit einem Polymerisationsgrad von 2 und größer von größer 60%, einen Gehalt an Polyglycerinen mit einem Polymerisationsgrad von 3 und größer von größer 50% und einen Gehalt an Polyglycerinen mit einem Polymerisationsgrad von 4 und größer von größer 40%.

*Synthesebeispiel 4), nicht erfindungsgemäß*

Synthesebeispiel 4a)

Ein Gemisch aus Glycerin (2102 g, 22,8 mol) und 45%iger wässriger Kalilauge (24,2 g) wurde bei 400 mbar innerhalb von 1 Stunde auf 240 °C erhitzt und das entstehende Wasser kontinuierlich abdestilliert. Sobald das Reaktionsgemisch einen Brechungsindex von $\geq$1.4810 erreicht hatte, wurde der Druck langsam auf 50 mbar gesenkt und weiter Wasser sowie überschüssiges Glycerin bei 240 °C abdestilliert, bis das verbleibende Gemisch eine Hydroxylzahl von 1150 mg KOH/g aufwies.

Ein Gemisch aus dem so erhaltenen Polyglycerin (114,6 g, 0,48 mol) und Stearinsäure und Palmitinsäure im Verhältnis 1:1 (212,8 g, 0,79 mol) wurde innerhalb von 3 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von $\leq$10 erreicht war.

Synthesebeispiel 4b)

Ein Gemisch aus Glycerin (2102 g, 22,8 mol) und 45%iger wässriger Kalilauge (24,2 g) wurde bei 400 mbar innerhalb von 1 Stunde auf 240 °C erhitzt und das entstehende Wasser kontinuierlich abdestilliert. Sobald das Reaktionsgemisch einen Brechungsindex von $\geq$1.4810 erreicht hatte, wurde der Druck langsam auf 50 mbar gesenkt und weiter Wasser sowie überschüssiges Glycerin bei 240 °C abdestilliert, bis das verbleibende Gemisch eine Hydroxylzahl von 1150 mg KOH/g aufwies.

Ein Gemisch aus dem so erhaltenen Polyglycerin (174,6 g, 0,73 mol) und Caprylsäure (125,4 g, 0,86 mol) wurde innerhalb von 2 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von $\leq$10 erreicht war.

Synthesebeispiel 4c)

Ein Gemisch eines Polyglycerinesters erhalten wie beschrieben in Beispiel 4a) (100 g) und eines Polyglycerinesters erhalten wie beschrieben in Beispiel 4b) (33,3 g) wurde auf 240 °C erhitzt und das Gemisch anschließend so lange bei dieser Temperatur gerührt und das entstehende Wasser kontinuierlich entfernt, bis eine Säurezahl von $\leq$1.0 erreicht war.

Der so erhaltene Polyglycerinester setzt nach seiner vollständigen Hydrolyse pro Mol Polyglycerinester
ca. 1,1 mol Stearinsäure und Palmitinsäure im Verhältnis 1:1 und
ca. 0,4 mol Caprylsäure frei und
ist dadurch gekennzeichnet, dass das nach vollständiger Hydrolyse des Polyglycerinesters freigesetzte Polyglycerin ein Massenverhältnis von Glycerin zu Diglycerin von kleiner 1,5 und einen Polymerisationsgrad von ca. 3 aufweist.

*Synthesebeispiel 5), nicht erfindungsgemäß*

Ein Gemisch aus Glycerin (2102 g, 22,8 mol) und 45%iger wässriger Kalilauge (24,2 g) wurde bei 400 mbar innerhalb von 1 Stunde auf 240 °C erhitzt und das entstehende Wasser kontinuierlich abdestilliert. Sobald das Reaktionsgemisch einen Brechungsindex von $\geq$1.4810 erreicht hatte, wurde der Druck langsam auf 50 mbar gesenkt und weiter Wasser sowie überschüssiges Glycerin bei 240 °C abdestilliert, bis das verbleibende Gemisch eine Hydroxylzahl von 1150 mg KOH/g aufwies.

Ein Gemisch aus dem so erhaltenen Polyglycerin (99,5 g, 0,41 mol) und Stearinsäure und Palmitinsäure im Verhältnis 1:1 (50,0 g, 0,19 mol) und Caprylsäure (50,0 g, 0,35 mol) wurde innerhalb von 3 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von $\leq$3.0 erreicht war.

Der so erhaltene Polyglycerinester setzt nach seiner vollständigen Hydrolyse pro Mol Polyglycerinester
ca. 0,5 mol Stearinsäure und Palmitinsäure im Verhältnis 1:1 und
ca. 0,9 mol Caprylsäure frei und
ist dadurch gekennzeichnet, dass das nach vollständiger Hydrolyse des Polyglycerinesters freigesetzte Polyglycerin ein Massenverhältnis von Glycerin zu Diglycerin von kleiner 1,5 und einen Polymerisationsgrad von ca. 3 aufweist.

*Synthesebeispiel 6), nicht erfindungsgemäß*

Ein Gemisch aus Glycerin (2102 g, 22,8 mol) und 45%iger wässriger Kalilauge (24,2 g) wurde bei 400 mbar innerhalb von 1 Stunde auf 240 °C erhitzt und das entstehende Wasser kontinuierlich abdestilliert. Sobald das Reaktionsgemisch einen Brechungsindex von ≥1.4810 erreicht hatte, wurde der Druck langsam auf 50 mbar gesenkt und weiter Wasser sowie überschüssiges Glycerin bei 240 °C abdestilliert, bis das verbleibende Gemisch eine Hydroxylzahl von 1150 mg KOH/g aufwies.

Ein Gemisch aus dem so erhaltenen Polyglycerin (180,1 g, 0,75 mol) und Stearinsäure und Palmitinsäure im Verhältnis 1:1 (234,7 g, 0,87 mol) wurde innerhalb von 3 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von ≤1.0 erreicht war.

Der so erhaltene Polyglycerinester setzt nach seiner vollständigen Hydrolyse pro Mol Polyglycerinester ca. 1,2 mol Stearinsäure und Palmitinsäure im Verhältnis 1:1 frei und

ist dadurch gekennzeichnet, dass das nach vollständiger Hydrolyse des Polyglycerinesters freigesetzte Polyglycerin ein Massenverhältnis von Glycerin zu Diglycerin von kleiner 1,5 und einen Polymerisationsgrad von ca. 3 aufweist.

*Synthesebeispiel 7), nicht erfindungsgemäß*

Ein Gemisch aus Glycerin (2102 g, 22,8 mol) und 45%iger wässriger Kalilauge (24,2 g) wurde bei 400 mbar innerhalb von 1 Stunde auf 240 °C erhitzt und das entstehende Wasser kontinuierlich abdestilliert. Sobald das Reaktionsgemisch einen Brechungsindex von ≥1.4810 erreicht hatte, wurde der Druck langsam auf 50 mbar gesenkt und weiter Wasser sowie überschüssiges Glycerin bei 240 °C abdestilliert, bis das verbleibende Gemisch eine Hydroxylzahl von 1150 mg KOH/g aufwies.

Ein Gemisch aus dem so erhaltenen Polyglycerin (180,1 g, 0,75 mol) und Caprylsäure (126,0 g, 0,87 mol) wurde innerhalb von 3 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von ≤1.0 erreicht war.

Der so erhaltene Polyglycerinester setzt nach seiner vollständigen Hydrolyse pro Mol Polyglycerinester ca. 1,2 mol Caprylsäure frei und

ist dadurch gekennzeichnet, dass das nach vollständiger Hydrolyse des Polyglycerinesters freigesetzte Polyglycerin ein Massenverhältnis von Glycerin zu Diglycerin von kleiner 1,5 und einen Polymerisationsgrad von ca. 3 aufweist.

*Beispiel 8): Emulsionsstabilität*

Alle Konzentrationen in den Anwendungsbeispielen sind in Gewichtsprozent angegeben. Zur Herstellung der Emulsionen wurden dem Fachmann bekannte übliche Homogenisierverfahren eingesetzt.

Die Herstellung der Emulsionen erfolgte daher typischerweise so, dass Öl- und Wasserphase auf 70 - 75°C erwärmt wurden. Anschließend wurde entweder die Ölphase in die Wassereingerührt oder Öl- und Wasserphase ohne Rühren zusammengegeben. Anschließend wurde mit einem geeigneten Homogenisator (z.B. Ultrathurrax) für ca. 1-2 Minuten homogenisiert.

Stabilisierende Polymere (z.B. Carbomere) werden bevorzugt als Öldispersion bei Temperaturen von 50 - 60°C in die Emulsion eingerührt. Anschließend wird kurz homogenisiert.

Zugabe weiterer Inhaltsstoffe (z.B. Konservierungsmittel, Wirkstoffe) erfolgte bevorzugt bei 40°C. Falls die Rezepturen mit organischen Säuren konserviert wurden, wurde der pH-Wert der Emulsionen auf ca. 5 eingestellt.

Diese Versuche sollen zeigen, dass die erfindungsgemäßen Polyglycerinester Vorteile in Bezug auf Emulsionsstabilität aufweisen. Als Repräsentanten für auf dem Stand der Technik beruhenden polglycerinbasierten O/W Emulgatoren wurden dabei verschiedene nicht erfindungsgemäße Emulgatoren aus den Beispielen 4 bis 7 gewählt.

Zur Überprüfung der Lagerstabilität der Emulsionen wurden diese drei Monate bei Raumtemperatur, 40°C und 45°C gelagert. Zur Überprüfung der Kältestabilität wurde außerdem ein Monat lang bei -5°C gelagert und drei Gefrier-Tau-Zyklen von 25°C/-15°C/25°C durchgeführt. Deutlich Veränderungen im Erscheinungsbild oder der Konsistenz sowie insbesondere Öl- oder Wasserabscheidungen wurden als Kriterien für Instabilität gewichtet.

Die folgende Formulierung zeigt, dass die erfindungsgemäßen Emulgatoren in den Rezepturen a, b und c im Vergleich zu den nicht erfindungsgemäßen Emulgatoren in den Rezepturen d, e und g Vorteile bei der Stabilisierung typischer kosmetischer Emulsionen aufweisen. Auch eine auf dem Stand der Technik beruhende Mischung f aus polyglycerinbasiertem O/W Emulgator analog nicht erfindungsgemäßem Synthesebeispiel 6 mit handelsüblichem Glycerylcaprylat ist nicht in der Lage, die Emulsion in der gewünschten Art und Weise zu stabilisieren.

| Rezeptur 1 | 1a | 1b | 1c | 1d | 1e | 1f | 1g |
|---|---|---|---|---|---|---|---|
| **Erfindungsgemäßer Emulgator nach Bsp. 1** | 3,0 | | | | | | |
| **Erfindungsgemäßer Emulgator nach Bsp. 2** | | 3,0 | | | | | |
| **Erfindungsgemäßer Emulgator nach Bsp. 3** | | | 3,0 | | | | |
| **Nicht erfindungsgemäßer Emulgator nach Bsp. 4** | | | | 3,0 | | | |
| **Nicht erfindungsgemäßer Emulgator nach Bsp. 5** | | | | | 3,0 | | |
| **Nicht erfindungsgemäßer Emulgator nach Bsp. 6** | | | | | | 1,5 | |
| **Nicht erfindungsgemäßer Emulgator nach Bsp. 7** | | | | | | | 3,0 |
| Glyceryl Caprylate [1] | | | | | | 1.5 | |
| Mandelöl | 6,0 | 6,0 | 6,0 | 6,0 | 6,0 | 6,0 | 6,0 |
| Caprylic/Capric Triglyceride [2] | 6,0 | 6,0 | 6,0 | 6,0 | 6,0 | 6,0 | 6,0 |
| Glycerin | 3,0 | 3,0 | 3,0 | 3,0 | 3,0 | 3,0 | 3,0 |
| Wasser | Ad 100 | Ad 100 | Ad 100 | Ad 100 | Ad 100 | Ad 100 | Ad 100 |
| Carbomer [3] | 0,2 | 0,2 | 0,2 | 0,2 | 0,2 | 0,2 | 0,2 |
| Ethylhexyl Palmitate [4] | 0,8 | 0,8 | 0,8 | 0,8 | 0,8 | 0,8 | 0,8 |
| NaOH 10%aq. | 0,6 | 0,6 | 0,6 | 0,6 | 0,6 | 0,6 | 0,6 |
| Phenoxyethanol | 0,8 | 0,8 | 0,8 | 0,8 | 0,8 | 0,8 | 0,8 |
| | | | | | | | |
| Emulsionsstabilität | OK | OK | OK | NOK glasig, starke Koales zenz | NOK Ölsepa ration bei Wärm elager ung | NOK Keine Emulsi onsbild ung | NOK Keine Emulsi onsbild ung |

1) Dermosoft GMCY (Dr. Straetmans GmbH)
2) TEGOSOFT CT (Evonik Industries AG)
3) TEGO Carbomer 141 (Evonik Industries AG)
4) TEGOSOFT OP (Evonik Industries AG)

Beispiel 9): Vergleich der antimikrobiellen Stabilisierung kosmetischer Emulsionen unter Verwendung eines herkömmlichen Konservierungsmittels in Kombination mit erfindungsgemäßen bzw. nicht erfindungsgemäßen Emulgatoren

| Rezeptur 2 | 2a | 2b | 2c | 2d |
|---|---|---|---|---|
| **Erfindungsgemäßer Emulgator nach Bsp. 1** | **3,0** | | | |
| **Erfindungsgemäßer Emulgator nach Bsp. 2** | | **3,0** | | |
| **Nicht erfindungsgemäßer Emulgator nach Bsp. 4** | | | **3,0** | |
| **Nicht erfindungsgemäßer Emulgator nach Bsp. 6** | | | | **3,0** |
| Mandelöl | 6,0 | 6,0 | 6,0 | 6,0 |
| Caprylic/Capric Triglyceride [2] | 6,0 | 6,0 | 6,0 | 6,0 |
| Glycerin | 3,0 | 3,0 | 3,0 | 3,0 |
| Wasser | ad 100 | ad 100 | ad 100 | ad 100 |
| Carbomer [3] | 0,2 | 0,2 | 0,2 | 0,2 |
| Ethylhexyl Palmitate[4] | 0,8 | 0,8 | 0,8 | 0,8 |
| NaOH 10%aq. | 0,6 | 0,6 | 0,6 | 0,6 |
| | | | | |
| Phenoxyethanol | 0,5 | 0,5 | 0,5 | 0,5 |

Ergebnisse der mikrobiologischen Belastungstests durchgeführt in Anlehnung an Kapitel 5.1.3 Prüfung auf ausreichende Konservierung des europäischen Arzneibuchs, 4. Ausgabe, Grundwerk 2002. Die Untersuchung wurde durchgeführt mit Rezeptur 2 für die aufgeführten Keime..

| Eingesetzte Keime | Erfindungsgemäßer Emulgator nach Bsp. 1 | Erfindungsgemäßer Emulgator nach Bsp. 2 | Nicht erfindungsgemäßer Emulgator nach Bsp. 4 | Nicht erfindungsgemäßer Emulgator nach Bsp. 6 |
|---|---|---|---|---|
| E. coli | OK | OK | OK | NOK |
| Cd. albicans | OK | OK | NOK | NOK |

Die erfindungsgemäßen Emulgatoren nach Bsp. 1 und 2 unterstützen die Wirksamkeit des Konservierungsmittels, damit eine ausreichende mikrobielle Stabilität für die aufgeführten Keime erzielt werden kann. Dieser Effekt ist für die nicht erfindungsgemäßen Emulgatoren nach den Synthesebeispielen 4 und 6 nicht nachweisbar.

Formulierungsbeispiele

Die folgenden Beispiele sollen zeigen, dass die erfindungsgemäßen Polyglycerinester in einer Vielzahl kosmetischer Formulierungen eingesetzt werden können.
Es ist darüber hinaus mit Hilfe der erfindungsgemäßen Polyglycerinester möglich, Pigmente oder Festkörper stabil in Emulsionspräparate einzuarbeiten.
Weiterhin zeigen die Beispiele die gute Kompatibilität mit typischen Emulgatoren, Coemulgatoren, Ölen, Verdickern und Stabilisatoren sowie die gute Verträglichkeit mit emulsionsbelastenden Inhaltsstoffen wie UV-Filtern, antimikrobiellen Wirkstoffen oder kosmetischen Wirkstoffen.
Die Beispiele sollen die vorliegende Erfindung verdeutlichen, ohne diese einzuschränken.
*Formulierungsbeispiel mit Verlauf der Keimzahlen über eine vierwöchige Lagerzeit basierend auf dem erfindungsgemäßen Emulgator nach Synthesebeispiel 1:*

| Rezeptur | 3 |
|---|---|
| **Erfindungsgemäßer Emulgator nach Bsp. 1** | 6,0 |
| Caprylic/Capric Triglyceride | 3,5 |
| Isopropyl Palmitate | 3,5 |
| C12-15 Alkyl Benzoate | 3,5 |
| Dimethicone | 3,5 |
| Glycerin | 10,0 |
| Acrylates/C10-30 Alkyl Acrylate Crosspolymer | 0,2 |
| Alcohol | 3,0 |
| Wasser | Ad 100 |
| Trisodium EDTA 20%aq. | 1,0 |
| pH-Wert (einstellen auf pH 6 mit NaOH/Citronensäure etc. | 6,0 |

Ergebnisse der mikrobiologischen Belastungstests durchgeführt gemäß DIN EN ISO 11930. Für diese Versuchsreihe wurden zusätzlich zu den in DIN EN ISO 11930 aufgeführten Testmikroorganismen weitere Spezies eingesetzt, um das Wirkspektrum tiefgehender zu evaluieren.

Bei der Bewertung wurden nicht die Bewertungskriterien nach DIN EN ISO 11930 für die Formulierung angewendet, sondern die gegen bestimmte Gruppen von Mikroorganismen vorhandene selektive antimikrobielle Wirkung wurde bewertet. Der Test besteht aus der Kontamination von Produktproben mit Mikroorgansimen (Bakterien, Hefen und Schimmelpilzen) sowie der Bestimmung der Lebendzellzahl (KBE/g) zu den definierten Testzeitpunkten 0d, 7d, 14d und 28d nach der Kontamination.

| Testkeim (-gemisch) | Keimzahl/g nach | | | |
|---|---|---|---|---|
| | 0d | 7d | 14d | 28d |
| Staph. aureus<br>Staph. epidermis | $2,7 \times 10^5$ | $< 1,0 \times 10^2$ | $< 1,0 \times 10^2$ | $< 1,0 \times 10^2$ |
| Ps. aueruginosa<br>Pseudomonas putida<br>Burkholderia cepacia<br>Escherichia coli<br>Enterobacter gergoviae<br>Serratia marcescens | $2,4 \times 10^5$ | $1,2 \times 10^3$ | $< 1,0 \times 10^2$ | $< 1,0 \times 10^2$ |
| Candida albicans<br>Candida parapsilosis<br>Candida guilliermondii | $2,2 \times 10^5$ | $< 1,0 \times 10^2$ | $< 1,0 \times 10^2$ | $< 1,0 \times 10^2$ |
| Aspergillus brasiliensis | $2,9 \times 10^5$ | $> 5,0 \times 10^4$ | $> 5,0 \times 10^4$ | $< 1,0 \times 10^2$ |

Dieses Beispiel zeigt, dass die konservierende Wirkung der verwendeten Menge Alkohol durch die Verwendung des erfindungsgemäßen Emulgators soweit gesteigert werden kann, dass die antimikrobielle Stabilität der Emulsion gewährleistet ist.

Creme basierend auf nachhaltigen Inhaltsstoffen

| Rezeptur 4 | 4a | 4b | 4c |
|---|---|---|---|
| **Erfindungsgemäßer Emulgator nach Bsp. 2** | 3,0 | 3,0 | 2,0 |
| Glyceryl Stearate SE | | 0,5 | |
| Polyglyceryl-3 Dicitrate/Stearate [5)] | | | 1,0 |

(fortgesetzt)

| Rezeptur 4 | 4a | 4b | 4c |
|---|---|---|---|
| Cetearyl Alcohol | 1,3 | 1,3 | 1,3 |
| Glyceryl Stearate | 1,2 | 1,2 | 1,2 |
| Avocadoöl | 6,0 | 6,0 | 6,0 |
| Caprylic/Capric Triglyceride | 8,5 | 8,5 | 8,5 |
| Oleyl Erucate [6] | 5,0 | 5,0 | 5,0 |
| Glycerin | 3,0 | 3,0 | 3,0 |
| Wasser | Ad 100 | Ad 100 | Ad 100 |
| Xanthan Gum [7] | 0,2 | 0,2 | 0,2 |
| NaOH 10%aq. (pH-Wert Einstellung auf 5,0) | q.s. | q.s. | q.s. |
| Benzyl Alcohol, Glycerin, Benzoic acid, Sorbic acid [8] | 0,5 | 0,5 | 0,5 |
| 5) TEGO Care PSC 3 (Evonik Industries AG) 6) TEGOSOFT OER (Evonik Industries AG) 7) Keltrol CG-SFT (CP Kelco) 8) Rokonsal BSB-N (Ashland) | | | |

Dünnflüssige Lotion basierend auf nachhaltigen Inhaltsstoffen. Mikrobielle Stabilisierung der Emulsion ohne die Verwendung von Stoffen, gelistet in Anhang 6 der Kosmetikverordnung.

| Rezeptur 5 | 5a | 5b | 5c |
|---|---|---|---|
| **Erfindungsgemäßer Emulgator nach Bsp. 1** | 3,0 | 2,0 | 2,0 |
| Polyglyceryl-10 Stearate [9] | | 1,0 | |
| Glyceryl Stearate Citrate [10] | | | 1,0 |
| Glyceryl Stearate | 0,5 | 0,5 | 0,5 |
| Stearyl Alcohol | 0,5 | 0,5 | 0,5 |
| Mandelöl | 5,0 | 5,0 | 5,0 |
| Isoamyl Cocoate [11] | 5,5 | 5,5 | 5,5 |
| Caprylic/Capric Triglyceride [2] | 5,5 | 5,5 | 5,5 |
| Glycerin | 4,0 | 4,0 | 4,0 |
| Wasser | Ad 100 | Ad 100 | Ad 100 |
| Xanthan Gum [7] | 0,5 | 0,5 | 0,5 |
| Ethanol | 10,0 | 10,0 | 10,0 |
| 9) Polyaldo 10-1-S (Lonza GroupAG) 10) AXOL C 62 (Evonik Industries AG) 11) TEGOSOFT AC (Evonik Industries AG) | | | |

Creme mit aktiven Wirkstoffen

| Rezeptur 6 | 6a | 6b | 6c |
|---|---|---|---|
| **Erfindungsgemäßer Emulgator nach Bsp. 2** | 3,0 | 2,0 | 2,5 |
| Sodium Stearoyl Glutamate [12] | | 1,0 | |
| Sodium Cetearyl Sulfate [13] | | | 0,5 |

(fortgesetzt)

| Rezeptur 6 | 6a | 6b | 6c |
|---|---|---|---|
| Glyceryl Stearate | 1,5 | 1,5 | 1,5 |
| Stearyl Alcohol | 1,0 | 1,0 | 1,0 |
| Caprylic/Capric Triglyceride [2] | 10,0 | 10,0 | 10,0 |
| Ethylhexyl Palmitate [4] | 8,3 | 8,3 | 8,3 |
| Triisostearin [14] | 2,0 | 2,0 | 2,0 |
| Wasser | Ad 100 | Ad 100 | Ad 100 |
| Hydrolyzed Hyaluronic Acid [15] | 0,1 | 0,1 | 0,1 |
| Glycerin | 3,0 | 3,0 | 3,0 |
| Carbomer [16] | 0,2 | 0,2 | 0,2 |
| NaOH 10%aq. | 0,6 | 0,6 | 0,6 |
| Terminalia Arjuna Bark Extract, Pentylene Glycol [17] | 2,0 | 2,0 | 2,0 |
| Phenoxyethanol | 0,5 | 0,5 | 0,5 |
| 12) Eumulgin SG (BASF SE) 13) Lanette E (BASF SE) 14) TEGOSOFT TIS (Evonik Industries AG) 15) HyaCare 50 (Evonik Industries AG) 16) TEGO Carbomer 134 (Evonik Industries AG) 17) TEGO Arjuna S (Evonik Industries AG) | | | |

Sonnenschutzlotion SPF 20

| Rezeptur 7 | 7a | 7b | 7c | 7d |
|---|---|---|---|---|
| **Erfindungsgemäßer Emulgator nach Bsp. 1** | 3,0 | 2,0 | 2,5 | 2,0 |
| Glyceryl Stearate Citrate [10] | | 1,0 | | 1,0 |
| Potassium Cetyl Phosphate | | | 0,5 | |
| Cetearyl Alkohol | 1,0 | 1,0 | 1,0 | 1,0 |
| Caprylic/Capric Triglyceride [2] | 2,8 | 2,8 | 2,8 | 1,5 |
| Oleyl Erucate [6] | 2,0 | 2,0 | 2,0 | 1,0 |
| C12-15 Alkyl Benzoate | | | | 3,0 |
| Ethylhexyl Salicylate | 5,0 | 5,0 | 5,0 | 5,0 |
| Titanium Dioxide; Diethylhexyl Carbonate; Polyglyceryl-6 Polyhydroxystearate [19] | 8,9 | 8,9 | 8,9 | 8,9 |
| Octocrylene | 8,0 | 8,0 | 8,0 | 8,0 |
| Bis-Ethylhexyloxyphenol Methoxyphenyl Triazine [20] | 1,5 | 1,5 | 1,5 | 1,5 |
| Butyl Methoxydibenzoylmethane | 1,7 | 1,7 | 1,7 | 1,7 |
| Tocopheryl Acetate | 0,5 | 0,5 | 0,5 | 0,5 |
| Glycerin | 3,0 | 3,0 | 3,0 | 3,0 |
| Wasser | Ad 100 | Ad 100 | Ad 100 | Ad 100 |
| Carbomer [3] | 0,2 | 0,2 | 0,2 | 0,2 |
| NaOH 10% aq. | 0,5 | 0,5 | 0,5 | 0,5 |

(fortgesetzt)

| Rezeptur 7 | 7a | 7b | 7c | 7d |
|---|---|---|---|---|
| Methylisothiazolinone, Methylparaben, Ethylparaben; Dipropylene Glycol [21] | 0,5 | 0,5 | 0,5 | 0,5 |

19) TEGO SUN TDEC 45 (Evonik Industries AG)
20) Tinosorb S (BASF SE)
21) Microcare MEM (Thor)

Natürliche Make-up Foundation

| Rezeptur 8 | 8a | 8b |
|---|---|---|
| **Erfindungsgemäßer Emulgator nach Bsp. 1** | 4,0 | 3,0 |
| Polyglyceryl-6 Stearate; Polyglyceryl-6 Behenate [22] | | 2,0 |
| Cetearyl Alcohol | 1,5 | 1,5 |
| Glyceryl Stearate | 1,5 | 1,5 |
| Myristyl Myristate [23] | 2,0 | 2,0 |
| Isoamyl Cocoate [11] | 6,0 | 6,0 |
| Decyl Cocoate [24] | 7,0 | 7,0 |
| Wasser | Ad 100 | Ad 100 |
| Glycerin | 1,0 | 1,0 |
| Magnesium Aluminum Silicate [25] | 0,8 | 0,8 |
| Xanthan Gum [7] | 0,2 | 0,2 |
| Titanium Dioxide [26] | 8,0 | 8,0 |
| Iron Oxides [27] | 0,9 | 0,9 |
| Iron Oxides [28] | 0,2 | 0,2 |
| Iron Oxides [29] | 0,4 | 0,4 |
| Iron Oxides [30] | 0,1 | 0,1 |
| Glycerin; Aqua; Sodium levulinate; Sodium anisate [31] | 1,5 | 1,5 |
| Glyceryl Caprylate [1] | 0,2 | 0,2 |
| Citric Acid 20%aq. | 0,5 | 0,5 |
| Cellulose [32] | 2,0 | 2,0 |

22) TEGO Care PBS 6 (Evonik Industries AG)
23) TEGOSOFT MM (Evonik Industries AG)
24) TEGOSOFT DC (Evonik Industries AG)
25) Veegum Ultra (Vanderbilt Minerals)
26) Kronos 1171 (Kronos Systems)
27) Sicovit Gelb 10E (Rockwood Pigments)
28) Sicovit Rot 30E (Rockwood Pigments)
29) Sicovit Braun 70E (Rockwood Pigments)
30) Sicovit Schwarz 80E (Rockwood Pigments)
31) Dermosoft 1388 ECO (Dr. Straetmans GmbH)
32) TEGO Feel Green (Evonik Industries AG)

Creme

| Rezeptur 9 | 9a | 9b | 9c |
|---|---|---|---|
| **Erfindungsgemäßer Emulgator nach Bsp. 3** | **3,0** | **2,5** | **2,0** |
| Cetearyl Glucoside | | **0,5** | |
| Polyglyceryl-6 Distearate | | | **1,0** |
| Glyceryl Stearate | 4,0 | 4,0 | 4,0 |
| Cetearyl Alcohol | 2,0 | 2,0 | 2,0 |
| Avocadoöl | 8,0 | 8,0 | 8,0 |
| Isopropyl Myristate [33] | 8,0 | 8,0 | 8,0 |
| Glycerin | 2,0 | 2,0 | 2,0 |
| Xanthan Gum [7] | 0,2 | 0,2 | 0,2 |
| Glycerin | 1,0 | 1,0 | 1,0 |
| Wasser | Ad 100 | Ad 100 | Ad 100 |
| Phenoxyethanol; Benzoic Acid; Dehydroacetic Acid [34] | 0,5 | 0,5 | 0,5 |
| NaOH 10%aq. (pH Einstellung auf 5,5) | q.s. | q.s. | q.s. |
| 33) TEGOSOFT M (Evonik Industries AG)<br>34) Rokonsal ND (Ashland) | | | |

Deodorant

| Rezeptur 10 | **10a** | **10b** | **10c** |
|---|---|---|---|
| **Erfindungsgemäßer Emulgator nach Bsp. 2** | 3,0 | 1,5 | 1,5 |
| Methylglucose Sesquistearate [35] | 1,0 | 1,5 | 1,5 |
| Diethylhexyl Carbonate [36] | 3,5 | 3,5 | 3,0 |
| PPG-14 Butyl Ether [37] | 3,5 | 3,5 | |
| Isopropyl Palmitate | | | 3,0 |
| Hydroxypropyl Starch Phosphate [38] | 4,0 | 4,0 | 4,0 |
| Wasser | Ad 100 | Ad 100 | Ad 100 |
| Polyglyceryl-3 Caprylate [39] | 0,5 | 0,5 | 0,5 |
| Phenoxyethanol | 0,7 | 0,7 | 0,7 |
| 35) TEGO Care PS (Evonik Industries AG)<br>36) TEGOSOFT DEC (Evonik Industries AG)<br>37) TEGOSOFT PBE (Evonik Industries AG)<br>38) Structure XL (AkzoNobel)<br>39) TEGO Cosmo P 813 (Evonik Industries AG) | | | |

Antitranspirant-Deodorant-Formulierung

| Rezeptur 11 | **11a** | **11b** | **11c** |
|---|---|---|---|
| **Erfindungsgemäßer Emulgator nach Bsp. 1** | 3,0 | 2,0 | 3,0 |
| Methylglucose Sesquistearate [35] | 1,0 | 1,5 | |
| Polyglyceryl-4 Laurate [40] | | 0,5 | |

(fortgesetzt)

| Rezeptur 11 | 11a | 11b | 11c |
|---|---|---|---|
| Steareth-2 [41] | | | 2,0 |
| Steareth-20 [42] | | | 1,0 |
| Caprylic/Capric Triglyceride [2] | 3,5 | 3,5 | |
| Isopropyl Myristate [33] | 3,5 | 3,5 | |
| PPG-15 Stearyl Ether [43] | | | 3,0 |
| Phenoxyethyl Caprylate [44] | | | 4,0 |
| Hydroxyethyl Cellulose [45] | 0,5 | 0,5 | |
| Wasser | Ad 100 | Ad 100 | Ad 100 |
| Aluminum Chlorohydrate (50%aq.) | 15,0 | 15,0 | 20,0 |
| Phenoxyethanol | 0,5 | 0,5 | 0,5 |

40) TEGO Care PL 4 (Evonik Industries AG)
41) TEGO Alkanol S 2 Pellets (Evonik Industries AG)
42) TEGO Alkanol S 20 P (Evonik Industries AG)
43) TEGOSOFT E (Evonik Industries AG)
44) TEGOSOFT XC (Evonik Industries AG)
45) Natrosol 250 HHR (Ashland Inc.)

**Patentansprüche**

1. Polyglycerinester, der nach seiner vollständigen Hydrolyse im Mittel (Zahlenmittel) pro Mol Polyglycerinester von 1,1 bis 4 Mol, bevorzugt von 1,2 bis 3 Mol, besonders bevorzugt 1,3 bis 2,7 Mol, mindestens einer Carbonsäure mit 8 bis 24 Kohlenstoffatomen freisetzt, **dadurch gekennzeichnet, dass** nach vollständiger Hydrolyse des Polyglycerinesters ein Polyglycerin erhalten wird, in dem das Massenverhältnis von Glycerin zu Diglycerin größer 1,5, bevorzugt größer 2, besonders bevorzugt größer 3 ist, **dadurch gekennzeichnet, dass** nach seiner vollständigen Hydrolyse im Mittel (Zahlenmittel) pro Mol Polyglycerinester von 0,1 bis 1,5 Mol, bevorzugt von 0,2 bis 1,0 Mol, besonders bevorzugt von 0,3 bis 0,7 Mol, mindestens einer ersten Carbonsäure mit 14 bis 24 Kohlenstoffatomen und von 0,5 bis 3,9 Mol, bevorzugt von 0,8 bis 2,5 Mol, besonders bevorzugt von 1,0 bis 2,0 Mol, mindestens einer zweiten Carbonsäure mit 6 bis 12 Kohlenstoffatomen, freigesetzt werden.

2. Polyglycerinester gemäß Anspruch 1, **dadurch gekennzeichnet, dass** das nach vollständiger Hydrolyse des Polyglycerinesters erhaltene Polyglycerin einen mittleren Polymerisationsgrad von 1,5 bis 10, bevorzugt von 1,7 bis 6, besonders bevorzugt von 2 bis 3,5, aufweist und mindestens 50 Gew.-%, bevorzugt mindestens 60 Gew.-%, Polyglycerine mit einem Polymerisationsgrad von 2 und größer, mindestens 40 Gew.-%, bevorzugt mindestens 50 Gew.-%, Polyglycerine mit einem Polymerisationsgrad von 3 und größer und mindestens 30 Gew.-%, bevorzugt mindestens 40 Gew.-%, Polyglycerine mit einem Polymerisationsgrad von 4 und größer enthält, wobei sich die Gewichtsprozente auf den gesamten Gehalt an Polyglycerin beziehen.

3. Polyglycerinester gemäß mindestens einem der vorherigen Ansprüche, **dadurch gekennzeichnet, dass** das Molverhältnis der nach vollständiger Hydrolyse des Polyglycerinesters erhaltenen ersten Carbonsäure zur zweiten Carbonsäure zwischen 1 zu 1,5 bis 1 zu 5,0, bevorzugt zwischen 1 zu 2,0 bis 1 zu 4,0, besonders bevorzugt zwischen 1 zu 2,5 und 1 zu 3,0, liegt.

4. Polyglycerinester gemäß mindestens einem der vorherigen Ansprüche, **dadurch gekennzeichnet, dass** die erste und zweite Carbonsäure ausgewählt ist aus linearen, gesättigten, unsubstituierten Carbonsäuren.

5. Polyglycerinester gemäß mindestens einem der vorherigen Ansprüche, **dadurch gekennzeichnet, dass** die erste Carbonsäure eine Mischung von Stearinsäure und Palmitinsäure und die zweite Carbonsäure Caprylsäure ist.

6. Verfahren zur Herstellung eines Polyglycerinesters umfassend die Verfahrensschritte

   A) Bereitstellen eines Polyglycerins, in dem das Massenverhältnis von Glycerin zu Diglycerin größer 1,5, bevorzugt größer 2, besonders bevorzugt größer 3 ist.
   B) Verestern mindestens eines Teils des Polyglycerins mit mindestens einer Carbonsäure mit 8 bis 24 Kohlenstoffatomen,
   wobei das molare Verhältnis der in Verfahrensschritt B) eingesetzten Carbonsäure zu in Verfahrensschritt A) eingesetztem Polyglycerin von 1,1 zu 1 bis 4 zu 1, bevorzugt von 1,2 zu 1 bis 3 zu 1, besonders bevorzugt 1,3 zu 1 bis 2,7 zu 1, beträgt, **dadurch gekennzeichnet, dass** in Verfahrensschritt B) mindestens eine erste Carbonsäure mit 14 bis 24 Kohlenstoffatomen und mindestens eine zweite Carbonsäure mit 6 bis 12 Kohlenstoffatomen eingesetzt wird,
   wobei das molare Verhältnis der in Verfahrensschritt B) eingesetzten ersten Carbonsäure zu in Verfahrensschritt A) eingesetztem Polyglycerin von 0,1 zu 1 bis 1,5 zu 1, bevorzugt von 0,2 zu 1 bis 1,0 zu 1, besonders bevorzugt von 0,3 zu 1 bis 0,7 zu 1, und das molare Verhältnis der in Verfahrensschritt B) eingesetzten zweiten Carbonsäure zu in Verfahrensschritt A) eingesetztem Polyglycerin von 0,5 zu 1 bis 3,9 zu 1, bevorzugt von 0,8 zu 1 bis 2,5 zu 1, besonders bevorzugt von 1,0 zu 1 bis 2,0 zu 1, beträgt.

7. Verfahren gemäß Anspruch 6, **dadurch gekennzeichnet, dass** das in Verfahrensschritt A) bereitgestellte Polyglycerin einen mittleren Polymerisationsgrad von 1,5 bis 10, bevorzugt von 1,7 bis 6, besonders bevorzugt von 2 bis 3,5, aufweist und
   mindestens 50 Gew.-%, bevorzugt mindestens 60 Gew.-%, Polyglycerine mit einem Polymerisationsgrad von 2 und größer,
   mindestens 40 Gew.-%, bevorzugt mindestens 50 Gew.-%, Polyglycerine mit einem Polymerisationsgrad von 3 und größer und
   mindestens 30 Gew.-%, bevorzugt mindestens 40 Gew.-%, Polyglycerine mit einem Polymerisationsgrad von 4 und größer enthält,
   wobei sich die Gewichtsprozente auf den gesamten Gehalt an Polyglycerin beziehen.

8. Verfahren gemäß Anspruch 6, **dadurch gekennzeichnet, dass** in Verfahrensschritt B) als die erste Carbonsäure eine Mischung von Stearinsäure und Palmitinsäure und als die zweite Carbonsäure Caprylsäure eingesetzt werden.

9. Verwendung des Polyglycerinesters gemäß mindestens einem der Ansprüche 1 bis 5 oder des Polyglycerinesters erhältlich nach einem Verfahren gemäß mindestens einem der Ansprüche 6 bis 8 als Emulgator.

10. Verwendung des Polyglycerinesters gemäß mindestens einem der Ansprüche 1 bis 5 oder des Polyglycerinesters erhältlich nach einem Verfahren gemäß mindestens einem der Ansprüche 6 bis 8 zur Herstellung von kosmetischen oder pharmazeutischen Formulierungen.

11. Emulgator enthaltend Polyglycerinester gemäß mindestens einem der Ansprüche 1 bis 5 oder des Polyglycerinesters erhältlich nach einem Verfahren gemäß mindestens einem der Ansprüche 6 bis 8.

12. Kosmetische oder pharmazeutische Formulierung enthaltend Polyglycerinester gemäß mindestens einem der Ansprüche 1 bis 5 oder Polyglycerinester erhältlich nach einem Verfahren gemäß mindestens einem der Ansprüche 6 bis 8.

13. Kosmetische oder pharmazeutische Formulierung gemäß Anspruch 12, **dadurch gekennzeichnet, dass** der Polyglycerinester in Mengen von 0,1 bis 10 Gew.% bezogen auf die Gesamtformulierung enthalten ist.

14. Kosmetische oder pharmazeutische Formulierung gemäß mindestens einem der Ansprüche 12 oder 13, zusätzlich enthaltend eine Substanz ausgewählt aus der Gruppe der Substanzen der kosmetischen Farbpigmente.

15. Kosmetische oder pharmazeutische Formulierung gemäß mindestens einem der Ansprüche 12 bis 14, **dadurch gekennzeichnet, dass** es sich um eine Antiperspirant und/oder Deodorant-Formulierung handelt.

16. Kosmetische oder pharmazeutische Formulierung gemäß mindestens einem der Ansprüche 12 bis 15, aufweisend einen pH-Wert von 3,0 bis 7,0, bevorzugt von 3,5 bis 6.

**Claims**

1. Polyglycerol ester which, after its complete hydrolysis, releases on average (number-average) per mole of polyglycerol ester from 1.1 to 4 mol, preferably from 1.2 to 3 mol, particularly preferably 1.3 to 2.7 mol, of at least one carboxylic acid having 8 to 24 carbon atoms,
**characterized in that**, following complete hydrolysis of the polyglycerol ester, a polyglycerol is obtained in which the mass ratio of glycerol to diglycerol is greater than 1.5, preferably greater than 2, particularly preferably greater than 3, **characterized in that**, after its complete hydrolysis, on average (number-average) per mole of polyglycerol ester from 0.1 to 1.5 mol, preferably from 0.2 to 1.0 mol, particularly preferably from 0.3 to 0.7 mol, of at least one first carboxylic acid having 14 to 24 carbon atoms and
from 0.5 to 3.9 mol, preferably from 0.8 to 2.5 mol, particularly preferably from 1.0 to 2.0 mol, of at least one second carboxylic acid having 6 to 12 carbon atoms are released.

2. Polyglycerol ester according to Claim 1,
**characterized in that** the polyglycerol obtained after complete hydrolysis of the polyglycerol ester has an average degree of polymerization of from 1.5 to 10, preferably from 1.7 to 6, particularly preferably from 2 to 3.5, and comprises at least 50% by weight, preferably at least 60% by weight, of polyglycerols with a degree of polymerization of 2 and greater,
at least 40% by weight, preferably at least 50% by weight, of polyglycerols with a degree of polymerization of 3 and greater and
at least 30% by weight, preferably at least 40% by weight, of polyglycerols with a degree of polymerization of 4 and greater,
where the percentages by weight refer to the total content of polyglycerol.

3. Polyglycerol ester according to at least one of the preceding claims, **characterized in that** the molar ratio of the first carboxylic acid obtained after complete hydrolysis of the polyglycerol ester to the second carboxylic acid is between 1:1.5 and 1:5.0, preferably between 1:2.0 and 1:4.0, particularly preferably between 1:2.5 and 1:3.0.

4. Polyglycerol ester according to at least one of the preceding claims, **characterized in that** the first and the second carboxylic acid is selected from linear, saturated, unsubstituted carboxylic acids.

5. Polyglycerol ester according to at least one of the preceding claims, **characterized in that** the first carboxylic acid is a mixture of stearic acid and palmitic acid and the second carboxylic acid is caprylic acid.

6. Process for producing a polyglycerol ester comprising the process steps

   A) provision of a polyglycerol in which the mass ratio of glycerol to diglycerol is greater than 1.5, preferably greater than 2, particularly preferably greater than 3.
   B) esterification of at least one part of the polyglycerol with at least one carboxylic acid having 8 to 24 carbon atoms,
   where the molar ratio of the carboxylic acid used in process step B) to polyglycerol used in process step A) is from 1.1:1 to 4:1, preferably from 1.2:1 to 3:1, particularly preferably 1.3:1 to 2.7:1, **characterized in that** in process step B) at least one first carboxylic acid having 14 to 24 carbon atoms and at least one second carboxylic acid having 6 to 12 carbon atoms are used, where the molar ratio of the first carboxylic acid used in process step B) to polyglycerol used in process step A) is from 0.1:1 to 1.5:1, preferably from 0.2:1 to 1.0:1, particularly preferably from 0.3:1 to 0.7:1, and the molar ratio of the second carboxylic acid used in process step B) to polyglycerol used in process step A) is from 0.5:1 to 3.9:1, preferably from 0.8:1 to 2.5:1, particularly preferably from 1.0:1 to 2.0:1.

7. Process according to Claim 6, **characterized in that** the polyglycerol provided in process step A) has an average degree of polymerization of from 1.5 to 10, preferably from 1.7 to 6, particularly preferably from 2 to 3.5, and comprises at least 50% by weight, preferably at least 60% by weight, of polyglycerols with a degree of polymerization of 2 and greater,
at least 40% by weight, preferably at least 50% by weight, of polyglycerols with a degree of polymerization of 3 and greater and

at least 30% by weight, preferably at least 40% by weight, of polyglycerols with a degree of polymerization 4 and greater,
where the percentages by weight refer to the total content of polyglycerol.

8. Process according to Claim 6, **characterized in that** in process step B) the first carboxylic acid used is a mixture of stearic acid and palmitic acid and the second carboxylic acid used is caprylic acid.

9. Use of the polyglycerol ester according to at least one of Claims 1 to 5 or the polyglycerol ester obtainable by a process according to at least one of Claims 6 to 8 as emulsifier.

10. Use of the polyglycerol ester according to at least one of Claims 1 to 5 or the polyglycerol ester obtainable by a process according to at least one of Claims 6 to 8 for producing cosmetic or pharmaceutical formulations.

11. Emulsifier comprising polyglycerol ester according to at least one of Claims 1 to 5 or the polyglycerol ester obtainable by a process according to at least one of Claims 6 to 8.

12. Cosmetic or pharmaceutical formulation comprising polyglycerol ester according to at least one of Claims 1 to 5 or polyglycerol ester obtainable by a process according to at least one of Claims 6 to 8.

13. Cosmetic or pharmaceutical formulation according to Claim 12, **characterized in that** the polyglycerol ester is present in amounts of from 0.1 to 10% by weight, based on the total formulation.

14. Cosmetic or pharmaceutical formulation according to at least one of Claims 12 and 13, additionally comprising a substance selected from the group of the substances of the cosmetic colour pigments.

15. Cosmetic or pharmaceutical formulation according to at least one of Claims 12 to 14, **characterized in that** it is an antiperspirant and/or deodorant formulation.

16. Cosmetic or pharmaceutical formulation according to at least one of Claims 12 to 15, having a pH of from 3.0 to 7.0, preferably from 3.5 to 6.

**Revendications**

1. Ester de polyglycérine, qui libère après son hydrolyse totale en moyenne (moyenne en nombre), par mole d'ester de polyglycérine, de 1,1 à 4 moles, de préférence de 1,2 à 3 moles, de manière particulièrement préférée de 1,3 à 2,7 moles, d'au moins un acide carboxylique de 8 à 24 atomes de carbone,
**caractérisé en ce qu'**après l'hydrolyse totale de l'ester de polyglycérine, une polyglycérine est obtenue, dans laquelle le rapport en masse entre la glycérine et la diglycérine est supérieur à 1,5, de préférence supérieur à 2, de manière particulièrement préférée supérieur à 3, **caractérisé en ce qu'**après son hydrolyse totale, en moyenne (moyenne en nombre), par mole d'ester de polyglycérine, de 0,1 à 1,5 mole, de préférence de 0,2 à 1,0 mole, de manière particulièrement préférée de 0,3 à 0,7 mole, d'au moins un premier acide carboxylique de 14 à 24 atomes de carbone, et
de 0,5 à 3,9 moles, de préférence de 0,8 à 2,5 moles, de manière particulièrement préférée de 1,0 à 2,0 moles, d'au moins un deuxième acide carboxylique de 6 à 12 atomes de carbone, sont libérés.

2. Ester de polyglycérine selon la revendication 1, **caractérisé en ce que** la polyglycérine obtenue après l'hydrolyse totale de l'ester de polyglycérine présente un degré de polymérisation moyen de 1,5 à 10, de préférence de 1,7 à 6, de manière particulièrement préférée de 2 à 3,5, et
contient au moins 50 % en poids, de préférence au moins 60 % en poids, de polyglycérines ayant un degré de polymérisation de 2 et plus,
au moins 40 % en poids, de préférence au moins 50 % en poids, de polyglycérines ayant un degré de polymérisation de 3 et plus, et
au moins 30 % en poids, de préférence au moins 40 % en poids, de polyglycérines ayant un degré de polymérisation de 4 et plus,
les pourcentages en poids se rapportant à la teneur totale en polyglycérine.

3. Ester de polyglycérine selon au moins l'une quelconque des revendications précédentes, **caractérisé en ce que** le rapport molaire entre le premier acide carboxylique et le deuxième acide carboxylique obtenus après l'hydrolyse totale de l'ester de polyglycérine est compris entre 1 sur 1,5 et 1 sur 5,0, de préférence entre 1 sur 2,0 et 1 sur 4,0, de manière particulièrement préférée entre 1 sur 2,5 et 1 sur 3,0.

**4.** Ester de polyglycérine selon au moins l'une quelconque des revendications précédentes, **caractérisé en ce que** le premier et le deuxième acide carboxylique sont choisis parmi les acides carboxyliques linéaires, saturés, non substitués.

**5.** Ester de polyglycérine selon au moins l'une quelconque des revendications précédentes, **caractérisé en ce que** le premier acide carboxylique est un mélange d'acide stéarique et d'acide palmitique, et le deuxième acide carboxylique est l'acide caprylique.

**6.** Procédé de fabrication d'un ester de polyglycérine comprenant les étapes de procédé suivantes :

A) la préparation d'une polyglycérine dans laquelle le rapport en masse entre la glycérine et la diglycérine est supérieur à 1,5, de préférence supérieur à 2, de manière particulièrement préférée supérieur à 3,
B) l'estérification d'au moins une partie de la polyglycérine avec au moins un acide carboxylique de 8 à 24 atomes de carbone,

le rapport molaire entre l'acide carboxylique utilisé à l'étape de procédé B) et la polyglycérine utilisée à l'étape de procédé A) étant de 1,1 sur 1 à 4 sur 1, de préférence de 1,2 sur 1 à 3 sur 1, de manière particulièrement préférée de 1,3 sur 1 à 2,7 sur 1, **caractérisé en ce qu'**à l'étape de procédé B), au moins un premier acide carboxylique de 14 à 24 atomes de carbone et au moins un deuxième acide carboxylique de 6 à 12 atomes de carbone sont utilisés, le rapport molaire entre le premier acide carboxylique utilisé à l'étape de procédé B) et la polyglycérine utilisée à l'étape de procédé A) étant de 0,1 sur 1 à 1,5 sur 1, de préférence de 0,2 sur 1 à 1,0 sur 1, de manière particulièrement préférée de 0,3 sur 1 à 0,7 sur 1, et le rapport molaire entre le deuxième acide carboxylique utilisé à l'étape de procédé B) et la polyglycérine utilisée à l'étape de procédé A) étant de 0,5 sur 1 à 3,9 sur 1, de préférence de 0,8 sur 1 à 2,5 sur 1, de manière particulièrement préférée de 1,0 sur 1 à 2,0 sur 1.

**7.** Procédé selon la revendication 6, **caractérisé en ce que** la polyglycérine préparée à l'étape de procédé A) présente un degré de polymérisation moyen de 1,5 à 10, de préférence de 1,7 à 6, de manière particulièrement préférée de 2 à 3,5, et
contient au moins 50 % en poids, de préférence au moins 60 % en poids, de polyglycérines ayant un degré de polymérisation de 2 et plus,
au moins 40 % en poids, de préférence au moins 50 % en poids, de polyglycérines ayant un degré de polymérisation de 3 et plus, et
au moins 30 % en poids, de préférence au moins 40 % en poids, de polyglycérines ayant un degré de polymérisation de 4 et plus,
les pourcentages en poids se rapportant à la teneur totale en polyglycérine.

**8.** Procédé selon la revendication 6, **caractérisé en ce qu'**à l'étape de procédé B), un mélange d'acide stéarique et d'acide palmitique est utilisé en tant que premier acide carboxylique, et l'acide caprylique est utilisé en tant que deuxième acide carboxylique.

**9.** Utilisation de l'ester de polyglycérine selon au moins l'une quelconque des revendications 1 à 5 ou de l'ester de polyglycérine pouvant être obtenu par un procédé selon au moins l'une quelconque des revendications 6 à 8 en tant qu'émulsifiant.

**10.** Utilisation de l'ester de polyglycérine selon au moins l'une quelconque des revendications 1 à 5 ou de l'ester de polyglycérine pouvant être obtenu par un procédé selon au moins l'une quelconque des revendications 6 à 8 pour la fabrication de formulations cosmétiques ou pharmaceutiques.

**11.** Émulsifiant contenant un ester de polyglycérine selon au moins l'une quelconque des revendications 1 à 5 ou un ester de polyglycérine pouvant être obtenu par un procédé selon au moins l'une quelconque des revendications 6 à 8.

**12.** Formulation cosmétique ou pharmaceutique contenant un ester de polyglycérine selon au moins l'une quelconque des revendications 1 à 5 ou un ester de polyglycérine pouvant être obtenu par un procédé selon au moins l'une quelconque des revendications 6 à 8.

**13.** Formulation cosmétique ou pharmaceutique selon la revendication 12, **caractérisée en ce que** l'ester de polyglycérine est contenu en quantités de 0,1 à 10 % en poids, par rapport à la formulation totale.

**14.** Formulation cosmétique ou pharmaceutique selon au moins l'une quelconque des revendications 12 ou 13, contenant en outre une substance choisie dans le groupe de substances des pigments colorés cosmétiques.

**15.** Formulation cosmétique ou pharmaceutique selon au moins l'une quelconque des revendications 12 à 14, **caractérisée en ce qu'**il s'agit d'une formulation d'antiperspirant et/ou de déodorant.

**16.** Formulation cosmétique ou pharmaceutique selon au moins l'une quelconque des revendications 12 à 15, présentant un pH de 3,0 à 7,0, de préférence de 3,5 à 6.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 2003104852 B
- JP 2010178723 B
- JP 2006055029 B
- EP 2363387 A
- DE 102008001788

**In der Beschreibung aufgeführte Nicht-Patentliteratur**

- **B. K. SCHRADER.** Grundlagen und Rezepturen der Kosmetika. Hüthig Buch Verlag Heidelberg, 329-341
- Kapitel 5.1.3 Prüfung auf ausreichende Konservierung des europäischen. Grundwerk, 2002